# EUROPEAN PATENT SPECIFICATION

(11) **EP 1 978 959 B1**
(45) Date of publication and mention of the grant of the patent: **09.11.2011**
(21) Application number: 06851953.7
(22) Date of filing: 18.09.2006
(51) Int. Cl.: A61K 31/47, C07D 211/22, C07D 211/26, C07D 405/06, C07D 295/13, C07D 295/096, C07D 317/58

(54) **PIPERIDINE AND PIPERAZINE DERIVATIVES**
PIPERIDIN- UND PIPERAZINDERIVATE
DÉRIVÉS DE PIPÉRIDINE ET DE PIPÉRAZINE

(30) Priority: 23.09.2005 US 720064 P
(43) Date of publication of application: 15.10.2008
(73) Proprietor: M's Science Corporation, Chuo-ku Kobe 650-0047 (JP)
(72) Inventor: SUN, Connie, L., Palo Alto, CA 94303 (US)
(74) Representative: Goodfellow, Hugh Robin
(86) International application number: PCT/IB2006/004324
(87) International publication number: WO 2008/096189

(56) References cited:
- EP-A- 0 711 763
- WO-A1-91/03243
- US-A- 5 281 598
- US-A- 5 736 546

## Description

### Background

This application claims priority based on U.S. provisional application No. 60/720,064 filed Sep. 23, 2005, entitled "Piperidine and Piperazine Derivatives," which is hereby incorporated herein by reference in the entirety of its disclosure.

The present invention relates to novel piperazine derivatives, to processes for preparing the novel derivatives, to novel intermediates useful in the process, to pharmaceutical compositions comprising the derivatives, and to the use of derivatives in the treatment of disorders of the central nervous system.

It has been disclosed in the scientific literature that certain disorders of the central nervous system may be treated using a modulator of sigma receptor function. Amongst compounds known to possess affinity for sigma ligands are certain piperidine and piperazine derivatives.

WO 91/09594 discloses compounds having affinity for sigma receptors, certain of which are piperidine or piperazine derivatives, and discloses that they are useful in the treatment of schizophrenia and other psychoses.

United States patent number 5,736,546 discloses certain 1,4-(diphenylalkyl)piperazines having one phenyl group unsubstituted and the other phenyl group substituted by two alkoxy groups. One of the compounds disclosed is 1-[2-(3,4-dimethoxyphenyl)ethyl]-4-(3-phenylpropyl)piperazine. It is also referred to in the scientific literature as SA 4503. The compounds of US 5,736,546 are said to be useful in the treatment of dementia, depression, schizophrenia, anxiety neurosis, diseases accompanying abnormal immune response, cryptorrhea and digestive ulcer.

WO 2004/110387 discloses that sigma ligands, in particular SA 4503, are also useful in the treatment of patients to facilitate neuronal regeneration after onset of a neurodegenerative disease, such as ischemic stroke, traumatic brain injury or spinal chord injury.

United States patent number 5,389,630 discloses certain diamine compounds having cerebral protective action. The compound of Example 50 is a piperazine derivative, but the vast majority of the exemplified compounds are homopiperazine derivatives. The mechanism of action of the compounds is not discussed.

EP 0 711 763 A discloses 1,4-di(phenylalkyl) piperazine derivatives, including SA 4503. US 5,281,598 discloses piperazine derivatives.

It has now been found that certain novel 1,4-piperidine and piperazine derivatives have high affinity for sigma receptors, in particular sigma-1 receptors.

According to one aspect, the present invention provides a compound of general formule (I) 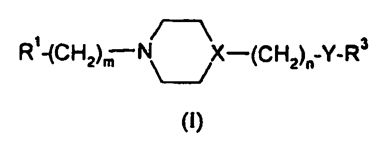 in which:-
R¹ represents a phenyl group that is substituted by one, two or three substituents selected independently from (1-2C)alkylenedioxy, a halogen atom, a hydroxyl group, a(1-4C) alkyl group, a (3-6C)cycloalkyl group, a halo(1-4C) alkyl group, a (1-4C)alkoxy group, a cyano group, and a halo(1-4C)alkoxy group;
m is 2, 3, or 4;
X is N;
n is 2, 3, or 4 or 5;
Y is O or NR² ;
R² is hydrogen, (1-4C)alkyl or phenyl(1-4C)alkyl, or is as defined for R³; and
R³ represents indan-1-yl, indan-2-yl, 1,2,3,4-tctrahydronaphth-1-yl or 1,2,3,4-tetrahydronaphth-2-yl, each of which may bear a hydroxyl substituent on a non-aromatic carbon atom; (3-6C) cycloalkyl; or a phenyl group that is unsubstituted or substituted by one, two or three substituents selected independently from (1-2C)alkylenedioxy, a halogen atom, a hydroxyl group, a (1-4C) alkyl group, a (3-6C)cycloalkyl group, a cyano group; a phenyl group, an imidazolyl group, a halo(1-4C) alkyl group, a (1-4C)alkoxy group and a halo(1-4C)alkoxy group;
or a pharmaceutically acceptable salt thereof.

Compounds according to the invention have been found to have high affinity for sigma receptors, in particular sigma-1 receptors.

As used herein, unless otherwise indicated, the term halogen atom includes fluorine, chlorine and bromine.

The term (1-2C)alkylenedioxy includes methylenedioxy and ethylenedioxy.

An example of a (1-4C) alkyl group is methyl. Other examples are ethyl, propyl, 2-propyl, butyl, 2-butyl and t-butyl.

The term halo(1-4C)alkyl as used herein includes perfluoro(1-4C)alkyl, such as trifluoromethyl.

An example of a (1-4C)alkoxy group is methoxy. Other examples are ethoxy, propoxy and 2-propoxy.

The term halo(1-4C)alkoxy as used herein includes pertluoro(1-4C)alkosy, such as trifluoromethoxy.

Examples of a (3-6C) cycloalkyl group are cyclopentyl and cyclohexyl.

Referring to formula (1), examples of particular values for R¹ are benzo[1,3]dioxol-5-yl, 2-fluorophenyl, 3-fluorophenyl, 4-fluorophenyl, 3,4-difluorophenyl, 3-chlorophenyl, 3-methylphenyl, 2-trifluoromethylphenyl, 3-trifluoromethylphenyl, 4-trifluoromethylphenyl, 2-methoxyphenyl, 3-methoxyphenyl, 4-methoxyphenyl, 4-isopropoxyphenyl, 3,4-dimethoxyphenyl, 2,3,4-trimethoxyphenyl, 3,4,5-trimethoxyphenyl, 2-fluoro-3,4-dimethoxyphenyl, 3-chloro-4-methoxyphenyl, 4-chloro-3-methoxyphenyl, 2-trifluoromethoxyphenyl, 4-trifluoromethoxyphenyl and 3-trifluoromethoxyphenyl.

Particular examples of values for R¹ are benzo[1,3]dioxol-5-yl, 2-fluorophenyl, 3,4-difluorophenyl, 2-trifluoromethylphenyl, 3-trifluoromethylphenyl, 4-trifluoromethylphenyl, 2-methoxypheny, 3-methoxyphenyl, 4-methoxyphenyl, 3,4-dimethoxyphenyl and 2-trifluoromethoxyphenyl.

Particular mention is made of compounds of formula (1) in which R¹ represents 3,4-dimethoxyphenyl, 4-methoxyphenyl, 4-isopropoxyphenyl, 4-trifluoromethoxyphenyl, or 3,4,5-trimethoxyphenyl.

Examples of values for m are 2 and 3. An example of a particular value for m is 2.

Examples of particular values for n are 2 and 3. An example of a particular value for n is 2.

An example of a particular value for R² is hydrogen.

Examples of particular values for Y are O and NH.

Examples of particular values for R³ are phenyl, benzo[1,3]dioxol-5-yl, 2-fluorophenyl, 4-fluorophenyl, 2,4-difluorophenyl, 4-trifluoromethoxyphenyl, 2-chlorophenyl, 4-chlorophenyl, 3,4-dichlorophenyl, 4-methylphenyl, 4-isopropylphenyl, 4-1-butylphenyl, 4-cyanophenyl, 2-trifluoromethylphenyl, 4-trifluoromethylphenyl, 2-methoxyphenyl, 4-methoxyphenyl, 4-biphenyl, 4-(1-imidazoly!)phenyl, 2-fluoro-4-methoxyptienyl, 3-fluoro-4-methoxyphenyl, 3-chloro-4-methoxyphenyl, 2-trifluoromethoxyphenyl, 3,4,5-trimethoxyphenyl, and 4-trifluoromethoxyphenyl.

Particular examples for R³ are phenyl, 2-fluorophenyl, 4-fluorophenyl, 2-chlorophenyl, 4-chlorophenyl, 4-methylphenyl, 4-isopropylphenyl, 4-cyanophenyl. 3,4,5-trimethoxygroup, and 2-trifluoromethylphenyl.

Particular mention is made of compounds of formula (I) in which R³ represents a 4-fluorophenyl group.

It will be appreciated that certain compounds of formula (I) contain a centre of asymmetry. These compounds may therefore exist and be isolated in the form of stereoisomers. The present invention provides a compound of formula (I) in any stereoisomeric form.

It will also be appreciated that the compounds of formula (I) or their pharmaceutically acceptable salts may be isolated in the form of a solvate, and accordingly that any such solvate is included within the scope of the present invention.

Certain compounds of formula (I) have also been found to possess good selectivity for sigma-1 receptors compared with sigma-2 receptors. This is particularly desirable, because the sigma-2 receptors have been shown to play an important role in the sigma receptor-mediated neck dystonia in rats (Matsumoto RR, et. al., Pharmacol. Biochem. Behav. 36, 151-155, 1996). For example microinjection of DTG (1,3-di-2-tolyl-guanidine, a sigma-1 and sigma-2 receptor agonist) induced neck dystonia in rats while injection of SA-4503 (a selective sigma I agonist) had no effect (Nakazawa M et. al., Pharmacol Biochem. Behav. 62, 123-126, 1999). In addition sigma-2 receptors have been implicated in the regulation of cell proliferation. Cytotoxic effects have been correlated with sigma-2 receptor ligands (Vilner and Bowen, Eur. J. Pharmacol Mol Pharmacol Sect 244, 199-201, 1993). Sigma-2 selective drugs can inhibit tumor cell proliferation through mechanisms that may involve apoptosis and intracellular calcium release (Aydar E et. al., Cancer Research 64, 5029-5035, 2004). These compounds are therefore particularly preferred.

According to another aspect, therefore, the present invention provides a compound which is selected from
1-(3,4-Dimethoxyphenethyl)-4-(2-(4-fluorophenoxy)ethyl)piperidine;
1-(4-(Trifluoromethyl)phenethyl)-4-(2-phenoxyethyl)piperidine;
4-(2-(2-Fluorophenoxy)ethyl)-1-(3,4-dimethoxyphenethyl)piperidine;
1-(3,4-Dimethoxyphenethyl)-4-(2-phenoxyethyl)piperazine;
1-(3-Methoxyphenethyl)-4-(2-phenoxyethyl)piperazine;
1-(4-Methoxyphenethyl)-4-(2-phenoxyethyl)piperazine;
1-(2-(Benzo[*d*][1,3]dioxol-5-yl)ethyl)-4-(2-phenoxyethyl)piperazine;
1-(3,4-Difluorophenethyl)-4-(2-phenoxyethyl)piperazine;
1-(3,4-Dimethoxyphenethyl)-4-(2-(4-chlorophenoxy)ethyl)piperazine ;
4-(2-(4-(3,4-Dimethoxyphenethyl)pipcrazin-1-yl)ethylo;y)benzonitrile;
and pharmaceutically acceptable salts thereof.

These compounds have both been found to possess good selectivity for sigma-1 over sigma-2 receptors.

The compounds of general formula (I) can be prepared by conventional processes.

According to another aspect, therefore, the present invention provides a process for preparing a compound of general formula (I), or a pharmaceutically acceptable salt thereof, which comprises
a) reducing a compound of general formula (II) 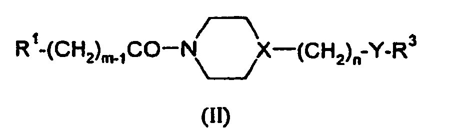 with a reducing agent;
b) for a compound of formula (I) in which X is N, reacting a compound of general formula (III) 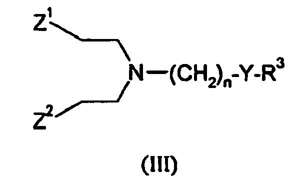 in which each of Z¹ and Z² independently represents a leaving atom or group, with a compound of general formula (IV)

   R¹-(CH₂)ₘ-NH₂ (IV)

   or a corresponding compound in which one or two substituents on R¹ are protected; or
c) for a compound of formula (I) in which X is N, reacting a compound of general formula (V) 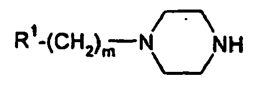 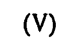 with a compound of general formula (VI)

   Z³-(CH₂)ₙ-Y-R³ (VI)

   in which Z³ represents a leaving atom or group;
   followed by removing any protecting group and, optionally, forming a pharmaceutically acceptable salt.

Referring to process step a), the reducing agent can conveniently be a borane (BH₃), a borohydride reducing agent, such as sodium borohydride, or an alkali metal aluminium hydride, such as lithium aluminium hydride. The reduction is conveniently performed in the presence of a solvent such as an ether, for example tetrahydrofuran. The.temperature at which the reduction is carried out is conveniently in the range of from -25 to 100 °C, such as from -10 to 40 °C.

Compounds of general formula (II) can be prepared by reacting a compound of general formula (VII) 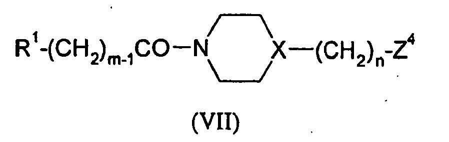 in which Z⁴ represents a leaving atom or group, such as a p-toluenesulfonyloxy group, with a compound of general formula (VIII)

H-Y-R³ (VIII).

Compounds of general formula (VII) can be prepared from a corresponding compound of general formula (IX), 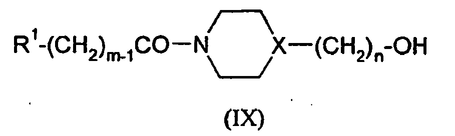 for example by reaction with a sulfonyl halide, such asp-toluenesulfonyl chloride.

Compounds of general formula (IX) can be prepared by reacting a compound of general formula (X) 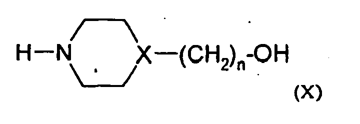 with a compound of general formula (XI)

R¹-(CH₂)ₘ₋₁COOH (XI)

or a reactive derivative thereof, using standard amide bond coupling conditions.

Alternatively, compounds of general formula (II) can be prepared by reacting a compound of general formula (XII) 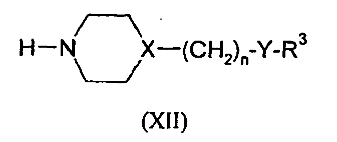 with a compound of general formula (XI), or a reactive derivative thereof, using standard amide bond coupling conditions.

Compounds of general formula (XII) can be prepared by deprotecting a compound of general formula (XIII) 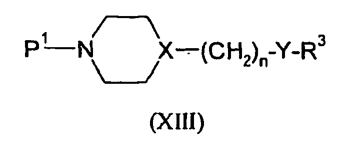 in which P¹ represents an amino protecting group, such as *t*-butoxycarbonyl.

Compounds of general formula (XIII) can be prepared from the corresponding compounds of general formula (XIV) 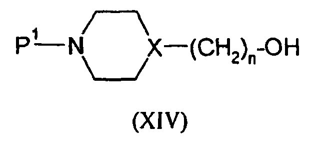 following the procedure for preparing a compound of general formula (II) from a compound of general formula (IX).

Referring to process step b), the leaving atoms or groups represented by Z¹ and Z² may be, for example, hydrocarbylsulfonyloxy groups, such as methanesulfonyloxy or p-toluenesulfonyloxy, or halogen atoms, such as chlorine atoms.

The reaction is conveniently performed at a temperature in the range of from 0 to 100°C, such as from 50 to 90 °C. Convenient solvents include organic solvents, for example amides such as dimethylformamide. The reaction is conveniently performed in the presence of a base, for example an alkali metal carbonate such as potassium carbonate. The reaction may be performed in the presence of a catalyst, such as sodium iodide.

Compounds of general formula (III) can be prepared from the corresponding compounds of general formula (XV) 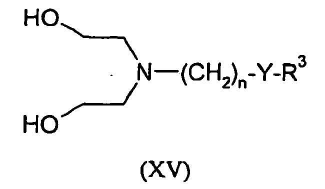 for example by reaction with thionyl chloride to afford a compound of formula (III) in which Z¹ and Z² represent chlorine atoms.

Compounds of general formula (XV) can be prepared by reacting a compound of general formula (XVI) 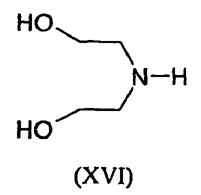 with a compound of general formula (XVII)

Z⁵-(CH₂)ₙ-Y-R³ (XVII)

in which Z⁵ represents a leaving atom or group, for example a halogen atom such as a bromine atom.

Referring to process step c), the leaving atom or group represented by Z³ may be, for example, a hydrocarbylsulfonyloxy group, such as *p*-toluenesulfonyloxy. Convenient solvents include ketones, such as acetone. The reaction is conveniently performed at a temperature in the range of from 0 to 100 °C.

A pharmaceutically acceptable salt may be formed by a conventional method, such as by reacting a compound of formula (I) with a pharmaceutically acceptable acid, such as hydrochloric acid.

Certain of the intermediates, for example compounds of formula (II), may be novel. The invention also provides the entire novel intermediates disclosed herein.

The compounds of the invention may be administered by any convenient route, e.g. into the gastrointestinal tract (e.g. rectally or orally), the nose, lungs, musculature or vasculature or transdermally. The compounds may be administered in any convenient administrative form, e.g. tablets, powders, capsules, solutions, dispersions, suspensions, syrups, sprays, suppositories, gels, emulsions, patches etc. Such compositions may contain components conventional in pharmaceutical preparations, e.g. diluents, carriers, pH modifiers, sweeteners, bulking agents, and further active agents. If parenteral administration is desired, the compositions will be sterile and in a solution or suspension form suitable for injection or infusion. Such compositions form a further aspect of the invention.

According to another aspect, the present invention provides a pharmaceutical composition, which comprises a compound of formula (I) or a pharmaceutically acceptable salt thereof, as defined hereinabove, together with a pharmaceutically acceptable diluent or carrier.

According to another aspect, the present invention provides the compound of formula (I), or a pharmaceutically acceptable salt thereof, for use in therapy.

According to another aspect, the present invention provides the use of the compound of formula (I), or a pharmaceutically acceptable salt thereof, for the , manufacture of a medicament for the treatment of a disorder responsive to a modulator of sigma receptor function.

According to another aspect, the present invention provides compounds for use in a method of treating a condition responsive to a modulator of sigma receptor function in a patient requiring treatment, wherein said method comprises administering to said patient an effective amount ofa compound of formula (I) or a pharmaceutically acceptable salt thereof.

The subject may be a human or a non-human animal, such as a non-human mammal, for example a cat, dog, horse, cow or sheep.

The disorder responsive to a sigma receptor modulator may be, for example, a disorder of the central nervous system, such as a neurological disorder or a psychiatric disorder that has been linked to sigma receptors. Examples of neurological disorders include cerebral deficits subsequent to cardiac bypass surgery and grafting, cerebral ischemia (e.g. associated with stroke or cardiac arrest); spinal cord trauma; head trauma; multiple sclerosis, Alzheimer's Disease; Huntington's Chorea; amyotrophic lateral sclerosis; AIDS-induced dementia; muscular spasms; convulsions; drug tolerance, withdrawal, and cessation (i.e. opiates, benzodiazepines, nicotine, cocaine, or ethanol); ocular damage and retinopathy; cognitive disorders; idiopathic and drug-induced Parkinson's Disease; pain; and movement disorders such as tardive dyskinesia. Examples of psychiatric disorders that are treated with a compound of formula I include schizophrenia, anxiety and related disorders (e.g. panic attack and stress-related disorders), depression, bipolar disorders, psychosis, and obsessive compulsive disorders.

The compounds according to the invention are of particular interest for use as neuroprotective agents and in the treatment of patients to facilitate neuronal regeneration and functional recovery after onset of a neurodegenerative disease, in particular ischemic stroke, traumatic brain injury, spinal chord injury and multiple sclerosis.

The dosage of the compounds of formula (I) will depend upon the nature and severity of the condition being treated, the administration route and the size and species of the subject. In general, quantities in the range of from 0.01 to 100 mg/kg bodyweight will be administered.

As used herein, the term "treatment" includes prophylactic use. The term "effective amount" refers to the amount of the compound of formula (I) that is effective to reduce or inhibit the development of the symptoms of the disorder being treated.

The compound according to the invention may be administered alone or in combination with another therapeutic agent having a different mode of action.

The ability of a compound to bind to a sigma receptor may be demonstrated by one or more of the following tests.

Sigma-1 (σ1) and sigma-2 (σ2) receptor binding assays are carried out in membranes from HEK-293 (Human Embryonic Kidney) cells.

### Membrane preparation:

Confluent HEK-293 cells are harvested in PBS/5 mM EDTA. They are centrifuged at 2000 rpm for 5 min and then washed two times in PBS. Cells are homogenized in 20 mM Tris-HCL (pH = 7.5) containing 5 mM EDTA, 0.5 mM PMSF and 0.5 µg/ml leupeptin using a Dounce homogenizer and sonicated for 5 minutes.

Nuclear debris and intact cells are removed by centrifugation at 3000 rpm for 10 minutes at 4 °C. The supernatant is centrifuged at 12000 rpm for 30 minutes and the resulting pellet is resuspended in 25 mM Tris-HCL (pH = 7.5) , 25 mM Mg₂Cl, 10% sucrose containing 0.5 mM PMSF, 2 mM AEBSF, I mM EDTA, 130 µM bestatin, 14 µM E-64, 1 µM leupeptin and 0.3 mM aprotinin.

Proteins are determined using the Bio Rad Protein Assay Dye Reagent and the membranes are aliquoted and frozen at -80°C.

### σ1 receptor-binding assay

The binding assays are performed in 96-well plates.

σ1 receptors are labeled using the σ1 selective probe (+)-(³H] Pentazocine (Bowen WD et al, Mol Neuropharmacol 3, 117-126, 1993).

Total binding is determined by incubating 50 µg of HEK-293 cell membranes with 10 nM (+)-[³H]-pentazocine (Perkin-Elmer, 35 Ci/mmol) and assay buffer (50 mM Tris-HCl, pH = 8.3) in a total volume of 200 µl. Non specific binding is determined in the presence of 10 µM unlabeled pentazocine. For competition experiments, 50 µl of displacing compound is added at 8 different concentrations. Incubations are carried out for 120 min at 37 °C. Assays are terminated by dilution with ice-cold 10 mM Tris-HCl, pH = 8.3 and vacuum filtration through glass fibers using a Skatron cell harvester from Molecular Devices. The filters are washed three times and the membrane-bound radioactivity is determined in a Microbeta scintillation counter.

Filters are soaked in 0.5 % polyethyleneimine for 1 hour before use.

Specific binding is determined by subtraction of non specific binding from total binding. IC₅₀ values (concentration of competing ligand required for 50% inhibition of [³H]-pentazocine binding) are analyzed by non-linear regression fit using the GraphPad Prism software.

### σ2 receptor binding assay

The binding assays are performed in 96-well plates

σ2 receptors are labeled using [³H] DTG (Di-o-tolylguanidine), under conditions in which σ1 receptors are masked with the σ1 selective compound pentazocine (Hellewell SB et al, Eur. J. Pharmacol, 268, 9-18, 1994).

Total binding is determined by incubating 50 µg of HEK-293 cell membranes with 10 nM [³H]-DTG (Perkin-Elmer, 58 Ci/mmol) in the presence of 10 µM pentazocine and assay buffer (50 mM Tris-HCl, pH = 8.3) in a total volume of 200 µl. Non specific binding is determined in the presence of 10 µM unlabeled DTG. For competition experiments, 50 µl of displacing compound is added at 8 different concentrations. Incubations are carried out for 120 min at 37 °C. Assays are terminated by dilution with ice-cold 10 mM Tris-HCl, pH = 8.3 and vacuum filtration through glass fibers using a Skatron cell harvester from Molecular Devices. The filters are washed three times and the membrane-bound radioactivity is determined in a Microbeta scintillation counter.

Filters are soaked in 0.5 % polyethyleneimine for 1 hour before use.

Specific binding is determined by subtraction of non specific binding from total binding. IC₅₀ values (concentration of competing ligand required for 50% inhibition of [³H]-DTG binding) are analyzed by non-linear regression fit using the GraphPad Prism software

The compounds exemplified herein have all been found to have an IC₅₀ of less than 700 nM in the σ1 receptor binding assay.

The following examples illustrate the invention. Examples marked "*" are provided for information, but are not embodiments of the invention.

### * Example 1

### 1-(3,4-Dimethoxyphenethyl)-4-(2-(4-chlorophenoxy)ethyl)piperidine

### Step 1: 2-(3,4-Dimethoxyphenyl)-1-[4-(2-hydroxyethyl)piperidin-1-yl]ethanone (A)

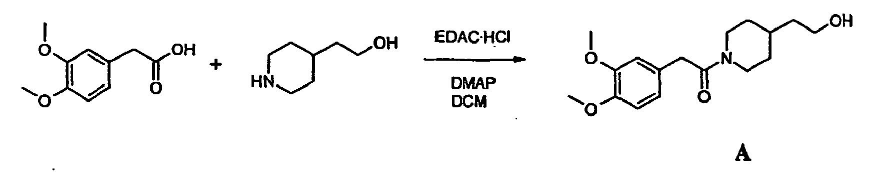

To an ice-cold solution of 3,4-dimethoxyphenylacetic acid (7.60 g, 38.7 mmol), *N,N-*dimethylaminopyridine (DMAP; 11.4 g, 93 mmol), and 4-piperidin-ethanol (5 g, 39 mmol) in dry dichloromethane (DCM; 80 mL) was added *N*-(3-dimethylaminopropyl)-*N*'-ethylcarbodiimide hydrochloride (EDAC-HCl; 9.65 g, 50.3 mmol) in one portion. The cooling bath was removed and the reaction was allowed to warm to room temperature. After five hours, HPLC analysis revealed the 3,4-dimethoxyphenylacetic acid was consumed. The reaction mixture was washed once with 1 N HCl_{(aq)} (90 mL) and concentrated *in vacuo.* The residue was chromatographed to yield the title compound (A; 10.67 g, 90% yield) as viscous oil.
¹H NMR (400 MHz, CDCl₃) 0.91 (br m, 1H), 1.04 (br m, 1H), 1.44 (q, *J*= 6.6 Hz, 2H), 1.64 (m, 3H), 1.73 (br s, 2H), 2.56 (br m, 1H), 2.92 (br m, 1H), 3.64 (m, 4H), 3.84 (s, 3H), 3.84 (s, 3H), 4.59 (br m, 1H), 6.73 (dd, *J*= 2.0, 8.2 Hz, 1H), 6.78 (d, *J* = 2.0 Hz, 1H), 6.79 (d, *J* = 8.2 Hz, 1H).
*m*/*z* 308 [M+1] ⁺.

### Step 2: Toluene-4-sulfonic acid 2-{1-[2-(3,4-dimethoxyphenyl)-acetyl]piperidin-4-yl}-ethyl ester (B)

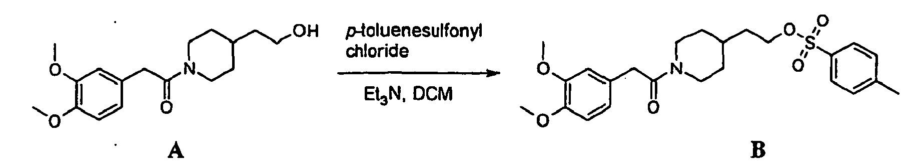

To a solution of **A** (10.67 g, 34.7 mmol) in dry DCM (100 mL) was added triethylamine (Et₃N; 7.5 mL, 53.8 mmol). The reaction mixture was cooled to 0 °C. *p-*Toluenesulfonyl chloride (10.0 g, 52.4 mmol) was added in one portion, and the mixture was stirred for five minutes. The cooling bath was removed and the reaction mixture was allowed to warm to room temperature as it stirred for 12 h. TLC analysis revealed the reaction to be nearly complete. The reaction mixture was washed once with 1 N HCl_{(aq)} (100 mL), concentrated *in vacuo,* and purified by chromatography to yield the title compound (**B**; 14.2 g, 88% yield) as an oil. *m*/*z* 462 [M+1]⁺.

### Step 3: 1-{4-[2-(4-Chlorophenoxy)ethyl]piperidin-1-yl}-2-(3,4-dimethoxyphenyl)-ethanone (C)

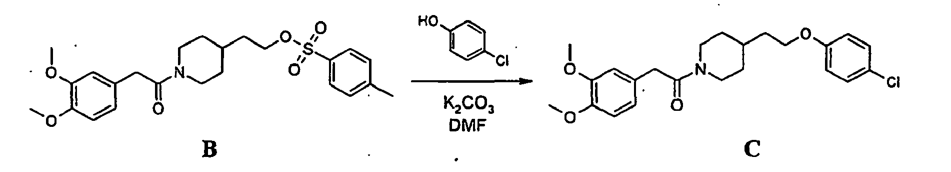

In a 20-mL reaction vessel equipped with a magnetic stir bar, **B** (1.4 g, 3.0 mmol), dry *N*,*N*-dimethylformamide (DMF; 11 mL), potassium carbonate (K₂CO₃; 1.26 g, 9.1 mmol) and 4-chlorophenol (0.78 g, 6.1 mmol) were heated at 75 °C for 15 h. The starting material was consumed by LC analysis. The reaction mixture was concentrated by heating under a nitrogen stream. The residue was dissolved in DCM (10 mL) and washed with water (10 mL). The organic layer was concentrated by heating under a nitrogen stream, and the residue was chromatographed to yield the title compound (**C**; 1.22 g, 96% yield) as an oil. *m*/*z* 418[M+1]⁺.

### Step 4: 1-(3,4-Dimethoxyphenethyl)-4-(2-(4-chlorophenoxy)ethyl)piperidine

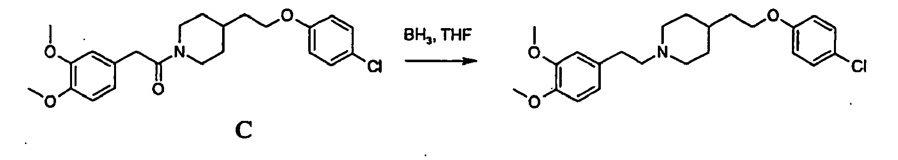

### Example 1

In a 20-ml reaction vessel equipped with a magnetic stir bar, C (1.22 g, 2.9 mmol) was dissolved in dry tetrahydrofuran (THF; 6.7 mL) with stirring. The solution was cooled to 0 °C, and 1.0 M borane (BH₃) solution in THF (8.8 mL, 8.8 mmol) was added slowly. After 10 minutes the cooling bath was removed, and the reaction mixture was stirred at room temperature for 12 h. LC analysis revealed complete consumption of C. Methanol was added slowly until gas evolution ceased (3-6 mL). The reaction mixture was concentrated by heating under a nitrogen stream. The residue was chromatographed to afford the title compound (0.17 g, 15%). ¹H NMR (400 MHz, DMSO-d₆): δ 1.5-1.9 (m, 7H), 2.6-3.0 (m, 8H), 3.71 (s, 3H), 3.74 (s, 3H), 4.01 (m, 2H), 6.75 (m, 1H), 6.81 (d, 1H), 6.88 (d, 1H), 6. 96 (m, 2H), 7.30 (m, 2H).
*m*/*z* 404 [M+1]⁺ .

The following compounds were prepared using the same method as described in Example 1.

### *Example 2

### 1-(3,4-Dimethoxyphenethyl)-4-(2-(4-fluorophenoxy)ethyl)piperidine

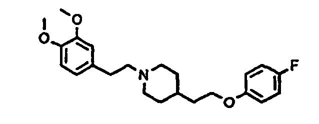

¹H NMR (400 MHz, DMSO-d₆): δ 1.47-1.7 (m, 8H), 2.7-3.1 (m, 7H), 3.72 (s, 3H), 3.75 (t, s, 3H), 3.97 (t, 2H), 6.76 (m, 1H), 6.82 (s, 1H), 6.86 (d, 1H), 6.94 (m, 2H), 7.09 (t, 2H).
*m*/*z* 388 [M+1]⁺.

### *Example 3

### 1-(3,4-Dimethoxyphenethyl)-4-(2-(p-tolyloxy)ethyl)piperidine

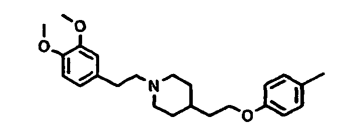

¹H NMR (400 MHz, CDCl₃): δ 1.4-1.9 (m, 7H), 2.20 (s, 3H), 2.6-3.0 (m, 8H), 3.69 (s, 3H), 3.72 (s, 3H), 3.95 (m, 2H), 6.73 (m, 1H), 6.78 (m, 3H), 6.85 (d, 1H), 7.04 (d, 2H).
*m*/*z* 384 [M+1]⁺.

### *Example 4

### 1-(3,4-Dimethoxyphenethyl)-4-(2-(4-isopropylphenoxy)ethyl)piperidine

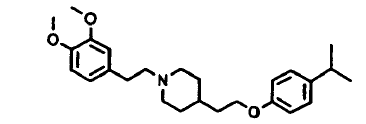

¹H NMR (400 MHz, DMSO-d₆): δ 1.13 (d, 6H), 1.5-1.9 (m, 8H), 2.6-3.0 (m, 8H), 3.69 (s, 3H), 3.73 (s, 3H), 3.94 (m, 2H), 6.72 (m, H), 4.06 (m, 2H), 6.72 (m, 1H), 6.80 (s, 1H), 6.85 (d, 1H), 6.90 (m, 1H), 6.09 (t, 1H), 7.16 (m, 2H).
*m*/*z* 388 [M+1]⁺.

### *Example 5

### 4-(2-(2-Fluorophenoxy)ethyl)-1-(3,4-dimethoxyphenethyl)piperidine

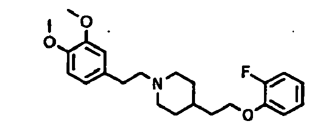

¹H NMR (400 MHz, CDCl₃): δ 1.4-1.9 (m, 8H), 2.6-3.0 (m, 7H), 3.69 (s, 3H), 3.72 (s, 3 H), 4.06 (m, 2H), 6.72 (m, 1H), 6.80 (s, 1H), 6.85 (d, 1H), 6.90 (m, 1H), 6.09 (t, 1H), 7.16 (m, 2H).
*m*/*z* 388 [M+1]⁺.

### *Example 6

### 4-(2-(2-Chlorophenoxy)ethyl)-1-(3,4-dimethoxyphenethyl)piperidine

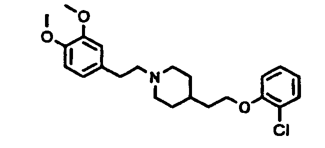

¹H NMR (400 MHz, DMSO-d₆): δ 1.54-1.89 (m, 7H), 2.58-2.66 (t, 1H), 2.80-3.03 (m, 6H), 3.71 (s, 3H), 3.74 (s, 3H), 4.09-4.13 (m, 2H), 6.72 (m, 1H), 6.82-6.90 (m, 2H), 6.93 (t, 1H), 7.12 (d, 1H), 7.27 (t, 1H), 7.38 (d, 1H).
*m*/*z* 404 [M+1]⁺.

### *Example 7

### 4-(2-(2-(Trifluoromethyl)phenoxy)ethyl)-1-(3,4-dimethoxyphenethyl)piperidine

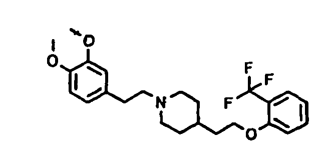

¹H NMR (400 MHz, CDCl₃): δ 1.4-1.9 (m, 8H), 2.6-3.0 (m, 7H), 3.69 (s, 3H), 3.72 (s, 3H), 4.14 (t, 2H), 6.73 (d, 1H), 6.83 (m, 2H), 7.06 (t, 1H), 7.24 (d, 1H), 7.59 (m, 2H).
*m*/*z* 438 [M+1]⁺.

### *Example 8

### 1-(3,4-Dimethoxyphenethyl)-4-(2-phenoxyethyl)piperidine

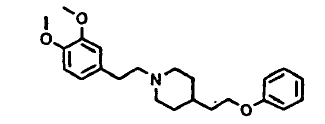

¹H NMR (400 MHz, DMSO-d₆): δ 1.4-1.9 (m, 8H), 2.6-3.0 (m, 7H), 3.69 (s, 3H), 3.72(s, 3H), 3.99 (m, 2H), 6.71 (t, 1H), 6.80 (s, 1H), 6.84-6.92 (m, 4H), 7.26 (m, 2H).
*m*/*z* 370 [M+1] ⁺.

### **Example 9

### 1-(2-(Benzo[d][1,3]dioxol-5-yl)ethyl)-4-(2-phenoxyethyl)piperidine

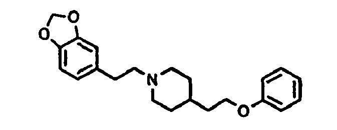

The title compound was prepared using a similar synthetic route as described in Example 1 but starting with methylenedioxyphenylacetic acid instead of 3,4-dimethoxyphenylacetic acid.
¹H NMR (400 MHz, CDCl₃): δ 1.5-1.99 (m, 8H), 2.7-3.0 (m, 7H), 3.98 (m, 2H), 5.95 (s, 2H), 6.66 (m, 1H), 6.79-6.81 (m, 2H), 6.91 (m, 3H), 7.25 (t, 2H) *m*/*z* 354 [M+1]⁺.

The following compounds can be prepared using the same procedure as described in Example 1:
4-[2-(4-Chloro-phenoxy)ethyl]-1-[2-(3,4-dimethoxyphenyl)ethyl]piperidine*
4-(2-{1-[2-(3,4-Dimedioxyphenyl)-ethyl)piperidin4-yl}ethoxy)benzonitrile*
1-[2-(3,4-Dimethoxyphenyl)ethyl]-4-[2-(4-methoxyphenoxy)ethyl]piperidine*
4-[2-(Biphenyl-4-yloxy)ethyl]-1-[2-(3,4-dimethoxyphenyl)ethyl]piperidine*
4-[2-(Benzo[1,3]dioxol-5-yloxy)ethyl]-1-[2-(3,4-dimethoxyphenyl)ethyl]piperidine*
1-[2-(3,4-Dimethoxyphenyl)ethyl]-4-[2-(4-trifluoromethylphenoxy)ethyl]piperidine*
1-[2-(3,4-Dimethoxyphenyl)ethyl]-4-[2-(4-imidazol-1-ylphenoxy)ethyl]piperidine*
1-[2-(3,4-Dimethoxyphenyl)ethyl]-4-[2-(4-trifluoromethoxyphenoxy)ethyl]piperidine*
1-[2-(3,4-Dimethoxyphenyl)ethyl]-4-[2-(2-methoxyphenoxy)ethyl]piperidine*
1-[2-(3,4-Dimethoxyphenyl)ethyl]-4-[2-(2-trifluoromethoxyphenoxy)ethyl]piperidine*
1-[2-(3,4-Dimethoxyphenyl)ethyl]-4-[2-(3-fluoro-4-methoxyphenoxy)ethyl]piperidine*
4-[2-(3-Chloro-4-methoxyphenoxy)ethyl]-1-[2-(3,4-dimethoxyphenyl)ethyl]piperidine* and
4-[2-(3,4-Dichlorophenoxy)ethyl]-1-[2-(3,4-dimethoxyphenyl)ethyl]piperidine.*

### *Example 10

### N-(2-(1-(3,4-Dimethogyphenethyl)piperidin-4-yl)ethyl)benzenamine

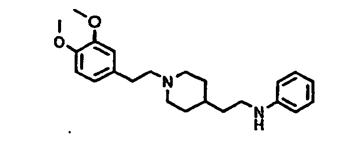

The title compound was prepared using the same procedure as described in Example 1 by replacing 4-chlorophenol with aniline in Step 3:
¹H NMR (400 MHz, CDCl₃): δ 1.4-1.9 (m, 8H), 2.6-3.0 (m, 9H), 3.69 (s, 3H), 3.72 (s, 3H), 5.35 (m, 1H), 6.47 (t, 1H), 6.51 (d, 2H), 6.73 9 (m, 1H), 6.80 (s, 1H), 6.85 (d, 1H), 7.03 (m, 2H).
*m*/*z* 369 [M+1]⁺.

The following compounds can be prepared using the same procedure as described in Example 10:
(2-{1-[2-(3,4-Dimethoxyphenyl)ethyl]piperidin-4-yl}ethyl)-(4-fluorophenyl)amine*
(4-Chlorophenyl)-(2-{1-[2-(3,4-dimethoxyphenyl)-ethyl]-piperidin-4-yl}ethyl)amine*
4-(2-{1-[2-(3,4-Dimethoxyphenyl)ethyl]piperidin-4-yl}ethylamino)benzonitrile*
(2-{1-[2-(3,4-Dimethoxyphenyl)ethyl]piperidin-4-yl}ethyl)-(4-methoxyphenyl)amine*
Biphenyl-4-yl-(2-{1-[2-(3,4-dimethoxyphenyl)ethyl]piperidin-4-yl}ethyl)amine*
Benzo[1,3]dioxol-5-yl-(2-{1-[2-(3,4-dimethoxyphenyl)ethyl]piperidin-4-yl}ethyl)amine*
(2-{1-[2-(3,4-Dimethoxyphenyl)ethyl]piperidin-4-yl}ethyl)-(4-trifluoromethylphenyl)-amine*
(2-{1-[2-(3,4-Dimethoxyphenyl)ethyl]piperidin-4-yl}ethyl)-*p*-tolylamine*
(2-{1-[2-(3,4-Dimethoxyphenyl)ethyl]piperidin-4-yl}ethyl)-(4-isopropylphenyl)amine*
(2-{1-[2-(3,4-Dimethoxyphenyl)ethyl]piperidin-4-yl}ethyl)-(4-imidazol-1-yl-phenyl)-amine*
(2-{1-[2-(3,4-Dimethoxyphenyl)ethyl]piperidin-4-yl}ethyl)-(4-trifluoromethoxyphenyl)amine*
(2-{1-[2-(3,4-Dimethoxyphenyl)ethyl]piperidin-4-yl}ethyl)-(2-fluorophenyl)amine*
(2-{1-[2-(3,4-Dimethoxyphenyl)ethyl]-piperidin-4-yl}ethyl)-(2-methoxyphenyl)amine*
(2-Chloro-phenyl)-(2-{1-[2-(3,4-dimethoxyphenyl)ethyl]piperidin-4-yl}ethyl)amine*
(2-{1-[2-(3,4-Dimethoxyphenyl)ethyl]-piperidin-4-yl}ethyl)-(2-trifluoromethylphenyl)-amine*
(2-{1-[2-(3,4-Dimethoxy-phenyl)ethyl]-piperidin-4-yl}ethyl)-(2-trifluoromethoxyphenyl)amine*
(2-{1-[2-(3,4-Dimethoxyphenyl)ethyl]piperidin-4-yl}ethyl)-(2-fluoro-4-methoxyphenyl)amine*
(2-{1-[2-(3,4-Dimethoxy-phenyl)ethyl]piperidin-4-yl}ethyl)-(3-fluoro-4-methoxyphenyl)amine*
(3-Chloro-4-methoxyphenyl)-(2-{1-[2-(3,4-dimethoxyphenyl)ethyl]piperidin-4-yl}-ethyl)amin* and
(3,4-Dichloro-phenyl)-(2-{1-[2-(3,4-dimethoxyphenyl)ethyl]piperidin-4-yl}ethyl)amine.*

### *Example 11

### 1-(2-Methoxyphenethyl)-4-(2-phenoxyethyl)piperidine

### Step 1: 4-[2-(Toluene-4-sulfonyloxy)ethyl]piperidine-1-carboxylic acid tert-butyl ester (D)

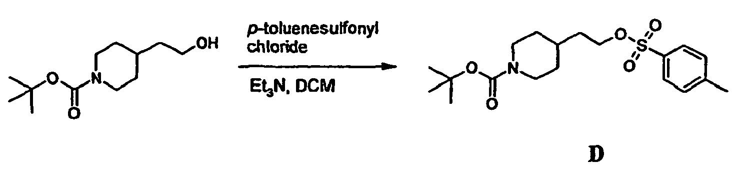

To an ice-cold solution of *N*-Boc-4-piperidine-ethanol (5.0 g, 21.8 mmol) and Et₃N (4.6 mL, 33.0 mmol) in dry DCM (10 mL) was added *p*-toluenesulfonyl chloride (6.25 g, 32.8 mmol) slowly. Upon addition, the reaction was warmed to room temperature and stirred for 20 h. The reaction mixture was washed with water (40 mL), 10% (wt/v) citric acid_{(aq)} (40 mL), and saturated NaHCO_{3(aq)} (40 mL). The DCM layer was dried with MgSO₄, filtered and concentrated *in vacuo* to give a pale yellow oil, which was chromatographed to give the title compound (7.47 g, 89% yield) as a clear and colorless oil.
¹H NMR (400 MHz, CDCl₃) 1.02 (m, 2 H), 1.43 (s, 9 H), 1.50-1.57 (m, 5H), 2.44 (s, 3H), 2.60 (br t, 2H), 4.02 (br d, 2H), 4.06 (t, 2H), 7.34 (d, 2H), 7.78 (d, 2H).

### Step 2: 4-(2-Phenoxy-ethyl)-piperidine-1-carboxylic acid tert-butyl ester (E)

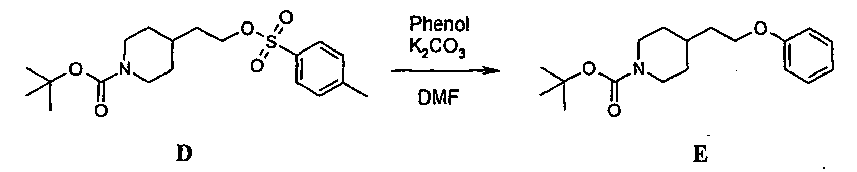

To a solution of **D** (8.24 g, 21.5 mmol) in dry DMF (85 mL) was added potassium carbonate (K₂CO₃; 8.9 g, 64 mmol) and phenol (4.0 g, 42.5 mmol). The reaction mixture was heated to 70-75 °C for 4 h, cooled to room temperature, and poured into water (300 mL). The mixture was extracted with ethyl acetate (3 x 100 mL). The ethyl acetate extracts were combined, washed with 0.25 M K₂CO_{3(aq)} (100 mL) and saturated sodium chloride (100 mL), dried over MgSO₄, and concentrated *in vacuo.* The resultant oil was run through a silica plug to remove very polar material, thus a mixture of the title compound and phenol (7.95 g) was isolated. This material was carried on to the next step without further purification. *m*/*z* 205 [M-CO₂C(CH₃)₃] ⁺.

### Step 3: 4-(2-Phenoxyethyl)-piperidine, hydrochloride salt (F)

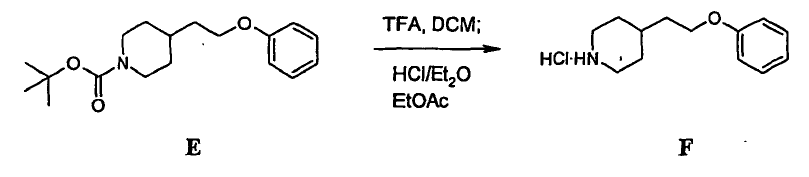

To a solution of **E** and phenol (7.95 g, 21.5 mmol **E** in theory) in DCM (80 mL) was added trifluoroacetic acid (20 mL) in a slow stream, and the mixture was stirred overnight at room temperature. The starting material was consumed by TLC analysis. The reaction mixture was washed with water (110 mL), and the aqueous phase was back-extracted with DCM (25 mL). The combined DCM layers were washed with saturated NaHCO_{3(aq)} (100 mL)), dried over MgSO₄, and concentrated to an oil (7.32 g). A portion of the oil (6.60 g) was dissolved in EtOAc (100 mL). With stirring 2 M HC! in Et₂O (Aldrich; 13.5 mL) was added dropwise. The mixture was stirred for 1.5 h, filtered, washed with EtOAc, and dried *in vacuo* to give F (4.0 g, 85% yield) as a white solid.
¹H NMR (400 MHz, DMSO-d₆) 1.39 (m, 2H), 1.67 (q, *J =* 6.4 Hz, 2H), 1.76 (m, 1H), 1.84 (br d, 2H), 2.83 (br q, *J*= 11.3 Hz, 2H), 3.22 (br d, *J* = 12.9 Hz, 2H), 4.00 (t, *J* = 6.3 Hz, 2H), 6.90-6.93 (m, 3H), 7.28 (m, 2H), 8.73 (br s, 1H), 8.96 (br s, 1H).

### Step 4: 2-(2-Methoxy-phenyl)-1-[4-(2-phenoxy-ethyl)piperidin-1-yl]ethanone (G)

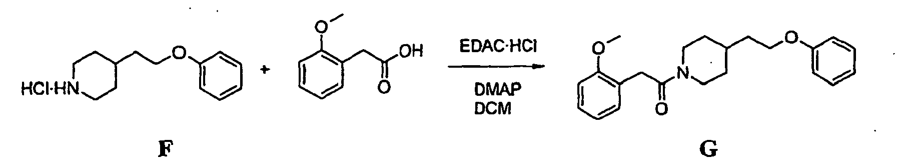

To a solution of F (0.32 g, 1.3 mmol), (2-methoxyphenyl)acetic acid (0.26 g, 1.6 mmol), and DMAP (0.55 g, 4.5 mmol) in DCM (6.5 mL) was added EDAC·HCl (0.36 g, 1.9 mmol). The reaction was stirred overnight at room temperature. HPLC analysis revealed complete consumption of F. The reaction mixture was washed with 1 N HCl(_{aq}) (2 x 5 mL), dried over MgSO₄, and concentrated by heating under a nitrogen stream to afford the title compound, which was carried on to the next step without further purification. *m*/*z* 354 [M +1)⁺.

### Step 5: 1-[2-(2-Methoxyphenyl)ethyl]-4-(2-phenoxyethyl)piperidine hydrochloride

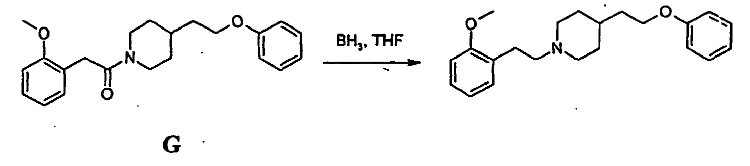

### Example 11

To an ice-cold solution of crude residue G (1:3 mmol in theory) in dry THF (3 mL) was added 1.0 M BH₃ in THF (Aldrich; 4.1 mL, 4.1 mmol) dropwise. Upon addition, the reaction mixture was warmed to room temperature and continued stirring for 19 h. HPLC analysis revealed the complete consumption of G. Methanol was added slowly until gas evolution ceased (1-3 mL). The reaction mixture was concentrated by heating under a nitrogen stream. The residue was chromatographed to afford the title compound (0.35 g, 79% from Step 4).
¹H NMR (400 MHz, DMSO-d₆): δ 1.5-1.7 (m, 8H), 2.8-3.0 (m, 7H), 3.77 (s, 3H), 3.98 (m, 2H), 6.84-6.95 (m, 5H), 7.13-7.27 (m, 4H).
*m*/*z* 3.40 [M + 1]⁺.

The following compounds were prepared using the same method as described in Example 11.

### *Example 12

### 1-(3-Methoxyphenethyl)-4-(2-phenoxyethyl)piperidine

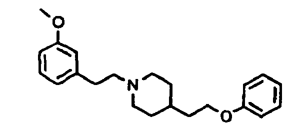

¹H NMR (400 MHz, CDCl₃): δ 1.40-1.80 (m, 7H), 2.10-2.40 (m, 1H), 2.48 (t, 1H), 2.89-3.18 (m, 6H), 3.81 (s, 3H), 4.02 (m, 2H), 6.76-6.97 (m, 6H), 7.21-7.31 (m, 3H).
*m*/*z* 340 [M+1]+.

### *Example 13

### 1-(4-Methoxyphenethyl)-4-(2-phenoxyethyl)piperidine

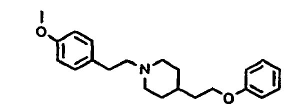

¹H NMR (400 MHz, DMSO-d₆): δ 1.40-1.90 (m, 9H), 2.70-3.10 (m, 6H), 3.70 (s, 3H), 3.98 (m, 2H), 6.84 (d, 2H), 6.90 (d, 3H), 7.13 (t, 2H), 7.23 (m, 2H).
*m*/*z* 340 [M + 1]⁺.

### *Example 14

### 1-Phenethyl-4-(2-phenoxyethyl)piperidine

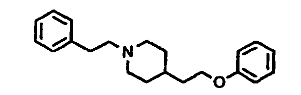

¹H NMR (400 MHz, DMSO-d₆): δ 1.5-1.9 (m, 9H), 2.8-3.1 (m, 6H), 4.01 (m, 2H), 6.91 (m, 3H), 7.2-7.3 (m, 7H).
*m*/*z* 310[M+1]⁺.

### *Example 15

### 1-(3-(Trifluoromethyl)phenethyl)-4-(2-phenoxyethyl)piperidine

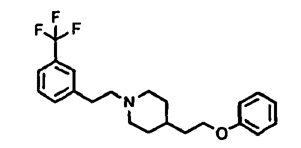

¹H NMR (400 MHZ, CDCl₃): δ 1.4-1.9 (m, 8H), 2.6-3.7 (m, 7H), 3.97 (m, 2H), 6.90 (m, 3H), 7.24 (m, 2H), 7.51-7.61 (m, 4H).
*m*/*z* 378 [M + 1]⁺.

### *Example 16

### 1-(4-(Trifluoromethyl)phenethyl)-4-(2-phenoxyethyl)piperidine

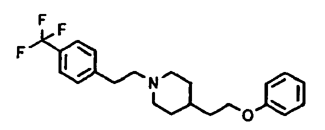

¹H NMR (400 MHz, DMSO-d₆): δ 1.4-1.7 (m, 8H), 2.6-3.1 (m, 7H), 3.97 (m, 2H), 6.90 (m, 3H), 7.25 (m, 2H), 7.47 (t, 2H), 7.64 (t, 2H).
*m*/*z* 378 [M + 1]⁺.

### *Example 17

### 1-(2-(Trifluoromethyl)phenethyl)-4-(2-phenoxyethyl)piperidine

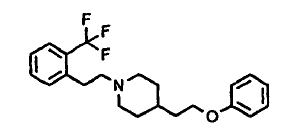

¹H NMR (400 MHz, CDCl₃): δ 1.4-1.7 (m, 8H), 2.7-3.2 (m, 7H), 3.97 (t, 2H), 6.91 (m, 3H), 7.25 (m, 2H), 7.44 (t, 1N), 7.49 (d, 1H), 7.63 (t, 1H), 7.68 (d, 1H).
*m*/*z* 378 [M + 1]⁺.

The following compounds can be prepared using the same procedures as for Example 11:
1-[2-(2-Fluoro-3,4-dimethoxyphenyl)ethyl]-4-(2-phenoxy-ethyl)piperidine*
4-(2-Phenoxyethyl)-1-[2-(2,3,4-trimethoxyphenyl)ethyl]piperidine*
1-[2-(2-Fluorophenyl)ethyl]-4-(2-phenoxyethyl)piperidine*
1-[2-(3-Fluorophenyl)ethyl]-4-(2-phenoxyethyl)piperidine*
1-[2-(4-Fluorophenyl)ethyl]-4-(2-phenoxyethyl)piperidine*
1-[2-(3,4-Difluorophenyl)ethyl]-4-(2-phenoxyethyl)piperidine*
4-(2-Phenoxyethyl)-1-(2-*m*-tolylethyl)piperidine*
1-[2-(3-Chlorophenyl)ethyl]-4-(2-phenoxyethyl)piperidine*
1-[2-(4-Chloro-3-methoxyphenyl)ethyl]-4-(2-phenoxyethyl)piperidine*
4-(2-Phenoxyethyl)-1-[2-(4-trifluoromethoxyphenyl)ethyl]piperidine*
4-[2-(4-Fluorophenoxy)ethyl]-1-[2-(4-methoxyphenyl)ethyl)piperidine*
4-[2-(4-Fluorophenoxy)ethyl]-1-[2-(4-trifluoromethory-phenyl)ethyl]piperidine*
4-[2-(2-Fluorophenoxy)ethyl]-1-[2-(4-methoxyphenyl)ethyl)piperidine*
4-[2-(2-Fluorophenoxy)ethyl]-1-[2-(4-trifluoromethoxyphenyl)ethyl] piperidine*
1-(2-Benzo[1,3]dioxol-5-ylethyl)-4-[2-(4-fluorophenoxy)ethyl]pipendine* and
1-(2-Benzo[1,3]dioxol-5-ylethyl)4-[2-(2-fluorophenoxy)ethyl]piperidine*.

### Example 18

### 1-(3,4-Dimethoxyphenethyl)-4-(2-phenoxyethyl)piperazine dihydrochloride

### Step 1: 2-[(2-Hydroxy-ethyl)-(2-phenoxy-ethyl)-amino]-ethanol (H)

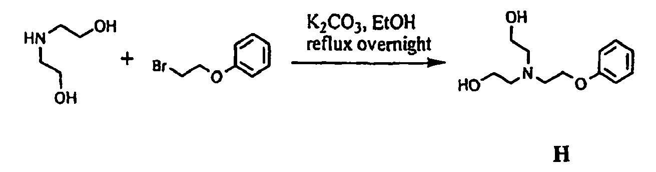

The reaction mixture of 2-(2-hydroxy-ethylamino)ethanol (32 g, 310 mmol), 1-(2-bromoethoxy)benzene (51 g, 256 mmol), and potassium carbonate (70 g, 512 mmol) in ethanol (200 mL) was refluxed overnight, concentrated, and partitioned between water and ethyl acetate. The organic layer was washed with brine, dried over anhydrous sodium sulfate, and concentrated. The residue was purified by column chromatography to afford the title compound (45 g, 78%).

### Step 2: Bis-(2-chloroethyl)-(2-phenoxyethyl)amine hydrochloride (I)

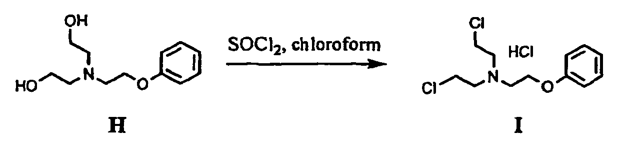

To an ice-cold solution of 2-[(2-hydroxyethyl)-(2-phenoxyethyl)-amino]ethanol (H; 43.9 g, 194 mmol) in chloroform (140 mL) was added thionyl chloride (SOCl₂; 114.8 g. 965 mmol) dropwise. The reaction mixture was then reflux for 1.5 h and concentrated. The residue was then suspended in the mixture of ethyl acetate and isopropyl ether. The precipitate was filtered and dried in a vacuum oven to afford the title compound quantitatively as a light brown crystal. This product was then used without further purification.

### Step 3: 1-(3,4-Dimethoxyphenethyl)-4-(2-phenoxyethyl)piperazine dihydrochloride

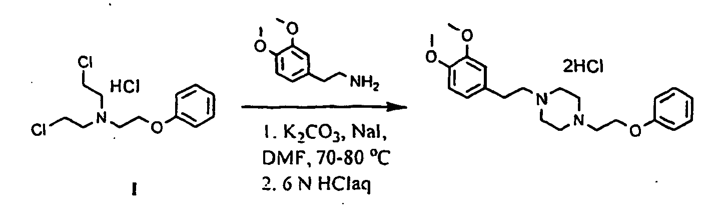

### Example 18

The reaction mixture of bis-(2-chloroethyl)-(2-phenoxyethyl)amine (0.895 g, 3 mmol), 2-(3,4-dimethoayphenyl)ethylamine (0.548 g, 3 mmol), potassium carbonate (K₂CO₃; 1.277 g, 9 mmol), and sodium iodide (Nal; 0.899 g, 6 mmol) in DMF (6 mL) was stirred at 70 - 80 °C for 5 h, quenched with water, and extracted with ethyl acetate. The organic layer was dried over anhydrous sodium sulfate and concentrated. The residue was suspended in 6 N HClaq (pH = 3-4) and filtered. The filter cake was then washed with ethyl acetate and ethanol and dried to afford the title compound as a pale white solid (330 mg, 23%). ¹H NMR (400 MHz, CD₃OD): δ 3.08 (m, 2H), 3.50 (m, 2H), 3.74-3.84 (m, 16H), 4.44 (t, 2H), 6.85-7.05 (m, 6H), 7.32 (m, 2H).
*m*/*z* 371 [M-2HC1+1]⁺.
The following compounds were prepared using the same method as described in Example 18.

### Example 19

### 1-(2-Methoxyphenethyl)-4-(2-phenoxyethyl)piperazine dihydrochloride

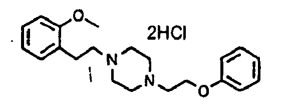

¹H NMR (400 MHz, CD₃OD): δ 3.13 (m, 2H), 3.46 (m, 2H), 3.5-3.9 (m, 13H), 4.45 (t, 2H), 6.90-7.06 (m, 5H), 7.24-7.35 (m, 4H).
*m*/*z* 341 [M-2HCl+1]⁺.

### Example 20

### 1-(3-Methoxyphenethyl)-4-(2-phenoxyethyl)piperazine dihydrochloride

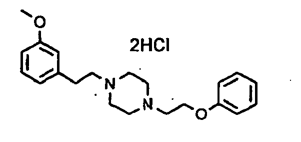

¹H NMR (400 MHz, CD₃OD): δ 3.12 (m, 2H), 3.52 (m, 2H), 3.65-3.95 (m, 13), 4.44 (t, 2H), 6.83-6.91 (m, 3H), 7.01-7.05 (m, 3H), 7.23-7.35 (m, 3H).
*m*/*z* 341 [M-2HCl+1)⁺.

### Example 21

### 1-(4-Methoxyphenethyl)-4-(2-phenoxyethyl)piperazine dihydrochloride

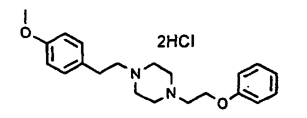

¹H NMR (400 MHz, CD₃OD): δ 3.08 (m, 2H), 3.48 (m, 2H), 3.6-3.0 (m, 13H), 4.45 (t, 2H), 6.90 (m, 2H), 7.03 (m, 3H), 7.23 (m, 2H), 7.32 (m, 2H).
*m*/*z* 341 [M-2HCl+1]⁺.

### Example 22

### 1-(2-(Benzo[d][1,3]dioxol-5-yl)ethyl)-4-(2-phenoxyethyl)piperazine dihydrochloride

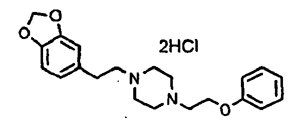

¹H NMR (400 MHz, CD₃OD): δ 3.05 (m, 2H), 3.46 (m, 2H), 3.6-3.9 (m, 10H), 4.42 (t, 2H), 5.93 (s, 2H), 6.38 (s, 2H), 6.84 (s, 1H), 7.01-7.04 (m, 3H), 7.32 (dd, 2H).
*m*/*z* 355 [M-2HCl+1)⁺.

### Example 23

### 1-(2-Fluorophenethyl)-4-(2-phenoxyethyl)piperazine dihydrochloride

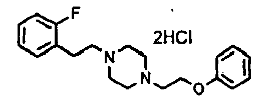

¹H NMR (400 MHz, CD₃OD): δ 3.21 (m, 2H), 3.50 (m, 2H), 3.6-3.9 (m, 1 0H), 4.39 (m, 2H), 6.98-7.21 (m, 5H), 7.30-7.40 (m, 4H).
*m*/*z* 329 [M-2HCl+1]⁺,

### Example 24

### 1-(3,4-Difluorophenethyl)-4-(2-phenoxyethyl)piperazine dihydrochloride

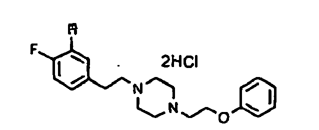

¹H NMR (400 MHz, CD₃OD): δ 3.14 (m, 2H), 3.51 9m, 2H), 3.6-3.9 (m, 10H), 4.43 (t, 2H), 6.98-7.05 (m. 3H). 7.06-7.35 (m, 5H).
*m*/*z* 347 [M-2HCl+1]⁺

### Example 25

### 1-(3-(Trifluoromethyl)phenethyn-4-(2-phenoxyethyl)piperazine dihydrochloride

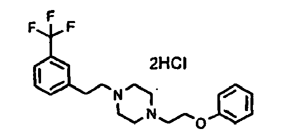

¹H NMR (400 MHz, CD₃OD): δ 3.25 (m, 2H), 3.54 (m, 2H), 3.7-3.9 (m, 10H), 4.45 (m, 2H), 7.02 (m, 3H), 7.34 (m, 2H), 7.55-7.69 (m, 4H).
*m*/*z* 379 [M-2HCl+1]⁺.

### Example 26

### 1-(2-(Trifluoromethoxy)phenethyl)-4-(2-pheaoxyethyl)piperazine dihydrochloride

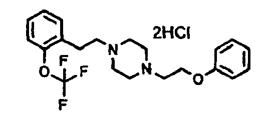

¹H NMR (400 MHz, CD₃OD+CDCl₃): δ 3.22 (m, 2H), 3.42 (m, 2H), 3.6-3.8 (m, 10H), 4.43 (t, 2H), 6.99 (m, 3H), 7.27-7.39 (m, 5H), 7.47 (d, 1H).
*m*/*z* 395 [M-2HCl+1)⁺.

### *Example 27

### 1-Phenethyl-4-(2-phenoxyethyl)piperazine dihydrochloride

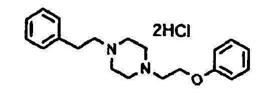

¹H NMR (400MHz, CD₃0D+CDCl₃): δ 3.15 (m. 2H), 3.48 (m, 2H), 3.7-4.0 (m, 10H), 4.43 (t, 2H), 6.99 (m, 3H), 7.25-7.34 (m, 7H).
*m*/*z* 311 [M-2HCl+1]⁺.

The following compounds can be prepared using the same procedure as described in Example 18:
1-[2-(2-Fluoro-3,4-dimethoxyphenyl)ethyl]-4-(2-phenoxyethyl)piperazine
1-(2-Phenoxyethyl)-4-[2-(2,3,4-trimethoxyphenyl)ethyl]piperazine
1-[2-(3-Fluorophenyl)ethyl]-4-(2-phenoxyethyl)piperazine
1-(2-(4-Fluorophenyl)-ethyl]4-(2-phenoxyethyl)piperazine
1-(2-Phenoxyethyl)-4-(2-*m*-tolylethyl)piperazine
1-(2-Phenoxyethyl)-4-[2-(2-trifluoromethyl-phenyl)ethyl]piperazine
1-(2-Phenoxyethyl)-4-[2-(4-trifluoromethylphenyl)ethyl]piperazine
1-(2-Phenoxyethyl)-4-[2-(3-trifluoromethoxyphenyl)ethyl]piperazine
1-[2-(3-Chloro-phenyl)ethyl]-4-(2-phenoxyethyl)piperazine and
1-[2-(3-Chloro-4-methoxyphenyl)ethyl]-4-(2-phenoxyethyl)piperazine.

### Example 28

### 1-(3,4-Dimethoxyphenethyl)-4-(2-(4-chlorophenoxy)ethyl)piperazine dihydrochloride

### Step 1: 2-(4-Chlorophenoxy)ethyl 4-methylbenzenesulfonate (J)

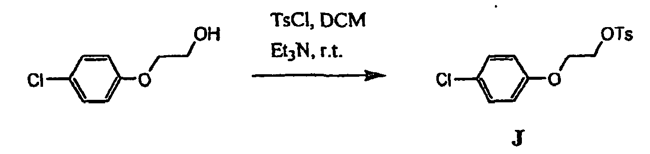

To a solution of 2-(4-chlorophenoxy)ethanol (2.0 g, 11.6 mmol) in DCM (10 mL) was added Et₃N (5 mL) and 4-methylbenzene-1-sulfonyl chloride (TsCl; 2.43 g, 12.8 mmol) sequentially. The reaction mixture was stirred at room temperature overnight and partitioned between water and ethyl acetate. The organic layer was washed with water, 5% sodium bicarbonate (NaHCO₃), and brine and concentrated. The resulting residue was washed with hexane to afford the title compound quantitatively.

### Step 2: 1-(3,4-Dimethoxyphenethyl)piperazine dihydrochloride (L) *

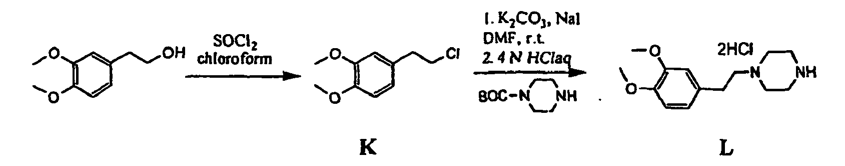

The reaction mixture of 2-(3,4-dimethoxyphenyl)ethanol (6.0 g, 32.8 mmol) and thionyl chloride (19 g, 164 mmol) in chloroform (20 mL) was stirred at reflux for 4 h, concentrated, and partitioned between water and ethyl acetate. The organic layer was washed with NaHCO₃ and brine, dried over anhydrous sodium sulfate, and concentrated to afford 4-(2-chloroethyl)-1,2-dimethoxybenzene (K) quantitatively (6.6 g).

A reaction mixture of 4-(2-chloroethyl)-1,2-dimethoxybenzene (3.6 g, 17.9 mmol), piperazine-1-carboxylic acid *t*-butyl ester (4.0 g, 21.5 mmol), K₂CO₃ (4.97 g, 36 mmol), and NaI (2.7 g, 18 mmol) in DMF (20 mL) was stirred at 80 °C for 3 h, cooled to room temperature, and partitioned between water and ethyl acetate. The organic layer was washed with water and brine, dried over anhydrous sodium sulfate, and concentrated. The residue was purified by column chromatography to afford 4-[2-(3,4-dimethoxyphenyl)-ethyl]-piperazine-1-carboxylic acid t-butyl ester (4.8 g, 76%) which was dissolved in 4 N HCl in dioxane. This solution was stirred at room temperature for 4 h, concentrated, and dried in a high vacuum oven to give the title compound quantitatively.

### Step 3: 1-(3,4-Dimethoxyphenethyl)-4-(2-(4-chlorophenoxy)ethyl)piperazine dihydrochloride

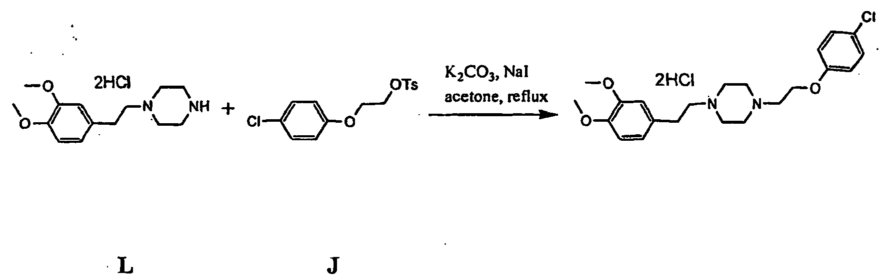

### Example 28

To the solution of 1-(3,4-dimethoxyphenethyl)piperazine dihydrochloride (502 mg, 1.55 mmol) and 2-(4-chlorophenoxy)ethyl 4-methylbenzenesulfonate (460 mg, 1.4 mmol) in acetone (20 mL) was added NaI (465 mg, 3.1 mmol) and k₂CO₃ (1.1 g, 7.0 mmol). The reaction mixture was then refluxed overnight and concentrated. The residue was partitioned between ethyl acetate and water. The organic layer was washed with water and brine, dried over anhydrous sodium sulfate, and concentrated. The resulting residue was purified by column chromatography to afford 1-(3,4-dimethoxyphenethyl)-4-(2-(4-chlorophenoxy)ethyl)piperazine which was suspended in 6 N HClaq. (pH = 3). The precipitate was filtered, washed with cold ethanol, and dried to afford the title compound (350 mg, 52%).
¹H NMR (400 MHz, DMSO-d₆): δ 2.96 (m, 2H), 3.4 (m, 12H), 3.70 (s, 3H), 3.74 (s, 3H), 4.36 (b, 2H), 6.76 (d, 1H), 6.89 (m, 2H), 7.03 (m, 2H), 7.34 (m, 2H).

### Example 29

### 1-(2-(2-Chlorophenoxy)ethyl)-4-(3,4-dimethoxyphenethyl)piperazine dihydrochloride

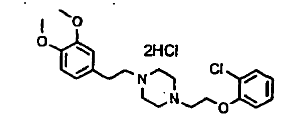

The title compound was prepared using the same procedure as described in Example 28 by replacing 2-(4-chlorophenoxy)ethyl 4-methylbenzenesulfonate with 2-(2-chlorophenoxy)ethyl 4-methylbenzenesulfonate in Step 1.
¹H NMR (400 MHz, DMSO-d₆): δ 2.96 (b, 2H), 3.4 (m, 12H), 3.70 (s, 3H), 3.72 (s, 3H), 4.44 (b, 2H), 6.76 (d, 1H), 6.89 (m, 2H), 6.98 (m, 1H), 7.20 (d, 1H), 7.33 (m, 1H), 7.46 (d, 1H).
*m*/*z* 405 [M-2HCl+1]⁺

### Example 30

### 4-(2-(4-(3,4-Dimethoxyphenethyl)piperazin-1-yl)ethyloxy)benzonitrile dihydrochloride

### Step 1: 4-(2-Bromoethoxy)benzonitrile (M)

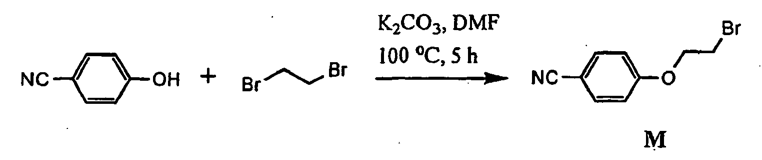

A reaction mixture of 4-hydroxybenzonitrile (1.19 g, 10 mmol), 1,2-dibromoethane (9.39 g, 50 mmol), and K₂CO₃ (4.14 g, 30 mmol) in DMF (20 mL) was stirred at 100 °C for 5 h and cooled to room temperature. To the reaction mixture was added ethyl acetate and water. The organic layer was washed with brine, dried over anhydrous sodium sulfate, and concentrated. The residue was purified by column chromatography to afford the 4-(2-bromoethoxy)benzonitrile (1.2 g, 53%).

### Step 2: 4-(2-(4-(3,4-Dimethoxyphenethyl)piperazin-1-yl)ethyloxy)benzonitrile dihydrochloride

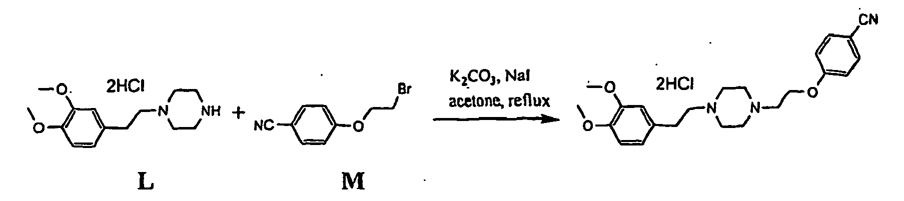

### Example 30

The title compound was prepared (4 1 % yield) using the same procedures as described in Example 28 (Step 3) by replacing 2-(4-chlorophenoxy)ethyl 4-methylbenzenesulfonate with 4-(2-bromoethoxy)benzonitrile.
¹H NMR (400 MHz, DMSO-d₆): δ 2.95-3.00 (m, 3H), 3.20-3.60 (m, 11H), 3.70 (s, 3H), 7.73 (s, 3H), 4.46 (b, 2H), 6.77 (d, 1H), 6.88 (d, 2H), 7.17 (d, 2H), 7.79 (d, 2H).
*m*/*z* 396 [M-2HC1+1]⁺.

The following compounds were prepared using the same procedure as described in Example 30 by replacing 4-hydroxybenzonitrile with the corresponding substituted phenol.

### Example 31

### 1-(3,4-Dimethoxyphenethyl)-4-(2-(4-isopropylphenoxy)ethyl)piperazine dihydrochloride

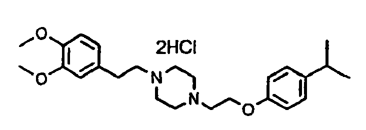

¹H NMR (400 MHz, CD₃OD): δ 1.20(d, 6H), 2.69 (s, 1H), 2.85 (m, 1H), 3.09 (m, 2H), 3.51 (m, 2H), 3.73-3.80 (m, 15H), 4.14 (m, 2H), 6.85-6.97 (m, 5H), 7.18 (q, 2H).
*m*/*z* 413 [M-2HCl+1]⁺.

### Example 32

### 1-(2-(2-(Trifluoromethyl)phenoxy)ethyl)-4-(3,4-dimethoxyphenethyl)piperazine dihydrochloride

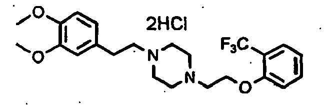

¹H NMR (400 MHz, CD₃OD): δ 3.09 (m, 2H), 3.51 (m, 2H), 3.6-4.0 (m, 16H), 4.62 (t, 2H), 6.85-6.96 (m, 3H), 7.16 (t, 1H), 7.26 (d, 1H), 7.63 (m, 2H).
*m*/*z* 439 [M-2HCl+1]⁺.

The following compounds can be prepared using the same procedure as described Example 28 and 30:
1-[2-(3,4-Dimethoxyphenyl)ethyl)4-[2-(4-fluoro-phenoxy)ethyl]piperazine
1-[2-(3,4-Dimethoxyphenyl)ethyl]-4-[2-(4-methoxyphenoxy)ethyl]piperazine
1-[2-(Biphenyl-4-yloxy)ethyl]-4-[2-(3,4-dimethoxyphenyl)ethyl]piperazine
1-(2-(Benzo[1,3]dioxol-5-yloxy)ethyl]-4-[2-(3,4-dimethoxyphenyl)ethyl]piperazine
1-[2-(3,4-Dimethoxy-phenyl)ethyl]-4-[2-(4-trifluoromethyl-phenoxy)ethyl]piperazine
1-[2-(3,4-Dimethoxy-phenyl)ethyl]-4-(2-*p*-tolyloxyethyl)piperazine
1-[2-(3,4-Dimethoxy-phenyl)ethyl]-4-[2-(4-imidazol-1-ylphenoxy)ethyl]piperazine
1-[2-(3,4-Dimethoxy-phenyl)ethyl]-4-[2-(4-trifluoromethoxyphenoxy)ethyl]piperazine
1-[2-(3,4-Dimethoxy-phenyl)ethyl]-4-[2-(2-fluorophenoxy)ethyl]piperazine
1-[2-(3,4-Dimethoxy-phenyl)ethyl]-4-[2-(2-methoxyphenoxy)ethyl]piperazine
1-[2-(3,4-Dimethoxy-phenyl)ethyl]-4-[2-(2-trifluoromethoxyphenoxy)ethyl]piperazine
1-[2-(3,4-Dimethoxy-phenyl)ethyl]-4-[2-(2-fluoro-4-methoxyphenoxy)ethyl]piperazine
1-[2-(3,4-Dimethoxy-phenyl)ethyl)-4-[2-(3-fluoro-4-methoxyphenoxy)ethyl]piperazine
1-[2-(3-Chloro-4-methoxy-phenoxy)ethyl]-4-[2-(3,4-dimethoxyphenyl)ethyl]piperazine
1-[2-(3,4-Dichloro-phenoxy)ethyl]-4-[2-(3,4-dimethoxyphenyl)ethyl]piperazine
1-[2-(4-Isopropoxy-phenyl)ethyl]-4-(2-phenoxy-ethyl)piperazine
1-[2-(4-Fluoro-phenoxy)ethyl]-4-[2-(4-isopropoxy-phenyl)ethyl]piperazine
1-[2-(2-Fluoro-phenoxy)ethyl]-4-[2-(4-isopropoxy-phenyl)ethyl]piperazine
1-[2-(2-Fluoro-phenoxy)ethyl]-4-[2-(3,4,5-trimethoxy-phenyl)ethyl]piperazine
1-[2-(2-Fluoro-phenoxy)ethyl]4-[2-(4-methoxy-phenyl)ethyl]piperazine
1-[2-(4-Fluoro-phenoxy)ethyl]-4-[2-(4-methoxy-phenyl)ethyl]piperazine
1-(2-Benzo[1,3]dioxol-5-ylethyl)-4-[2-(4-fluoro-phenoxy)ethyl]piperazine
1-(2-Phenoxyethyl)-4-[2-(4-trifluoromethoxyphenyl)ethyl]piperazine
4-(2-{4-[2-(4-Trifluoromethoxyphenyl)ethyl]piperazin-1-yl}ethoxy)benzonitrile
1-[2-(4-Fluoro-phenoxy)ethyl]-4-[2-(4-trifluoromethoxy-phenyl)ethyl]piperazine
1-[3-(3,4-Dimethoxy-phenyl)propyl]-4-(2-phenoxyethyl)piperazine
1-[3-(3,4-Dimethoxy-phenyl)propyl]-4-[2-(4-fluorophenoxy)ethyl]piperazine
1-(3-Benzo[1,3]dioxol-5-ylpropyl)-4-(2-phenoxyethyl)piperazine
1-(3-Benzo[1,3]dioxol-5-ylpropyl)-4-[2-(4-fluorophenoxy)ethyl]piperazine
1-[2-(4-Fluoro-phenoxy)ethyl]-4-[3-(4-methoxyphenyl)propyl]piperazine
1-[2-(4-Fluoro-phenoxy)ethyl]-4-[3-(4-isopropoxyphenyl)propyl)piperazine
1-[2-(4-Fluoro-phenoxy)ethyl)-4-[3-(4-trifluoromethoxyphenyl)propyl]piperazine
1-[2-(4-Fluoro-phenoxy)ethyl]-4-[3-(3,4,5-trimethoxyphenyl)propyl]piperazine and
1-[2-(4-*t*-Butyl-phenoxy)ethyl]-4-[2-(3,4-dimethoxyphenyl)ethyl]piperazine.

## Claims

1. A compound of general formula (I) 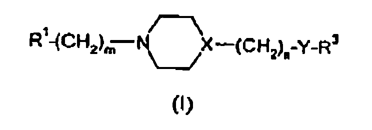 in which:
R¹ represents a phenyl group that is substituted by one, two or three substituents selected from the group consisting of (I-2C)atkylenedioxy, a halogen atom, a hydroxyl group, a cyano group, a (1-4C) alkyl group, a (3-6C)cycloalkyl group, a halo(1-4C) alkyl group, a (1-4C)alkoxy group, and a halo(1-4C)alkoxy group;
m is 2, 3 or 4;
X is N;
n is 2, 3 or 4;
Y is O or NR²;
R² is hydrogen, (1-4C)alkyl or phenyl(1-4C)alkyl, or is as defined for R³; and
R³ represents indan-1-yl, indan-2-yl, 1,2,3,4-terahydronaphth-1-yl, or 1,2,3,4-tetrahydronaphth-2-yl, each of which may bear a hydroxyl substituent on a non-aromatic carbon atom; a (3-6C) cycloalkyl group; or a phenyl group that is unsubstituted or substituted by one, two, or three substituents selected from the group consisting of (1-2C)alkylenedioxy, a halogen atom, a hydroxyl group, a (1-4C) alkyl group, a (3-6C)cycIoalkyl group, a cyano group; a phenyl group, an imidazolyl group, a halo(1-4C) alkyl group, a (1-4C)atkoxy group, and a halo(1-4C)alkoxy group;
or a pharmaceutically acceptable salt thereof.

2. A compound as recited in Claim 1, in which R¹ represents benzo[1,3]dioxol-5-yl. 2-fluorophenyl, 3-fluorophenyl, 4-fluorophenyl, 3,4- difluorophenyl, 3-chlorophenyl, 3-methylphenyl, 2-trifluoromethylphenyl, 3- trifluoromethylphenyl, 4-trifluoromethylphenyl, 2-methoxyphenyl, 3-methoxyphenyl, 4- methoxyphenyl, 4-isopropoxyphenyl, 3,4-dimethoxyphenyl, 2,3,4-trimethoxyphenyl, 3,4,5-trimethoxyphenyl, 2-fluoro-3,4-dimethoxyphenyl, 3-chloro-4-methoxyphenyl, 4- chloro-3-methoxyphenyl, 2-uifluoromahoxyphenyl, 4-trifluoromethoxyphenyl, or 3-trifluoromethoxyphenyl.

3. A compound as recited in Claim 1, in which R¹ represents benzo[1,3]dioxol-5-yl, 2-fluorophenyl, 3,4-difluorophonyl, 2-trifluoromethylphenyl, 3-txifluoromethylphenyl, 4-tifluorometlxylphenyl, 2-methoxyphenyl, 3-methoxyphenyl, 4-methoxyphenyl, 3,4-dimethoxyphenyl or 2- trifluoromethoxyphenyl.

4. A compound as recited in Claim 1, in which R¹ represents 3,4-dimethoxyphenyl, 3,4,5-trimethoxyphenyl, 4-methoxyphenyl, 4-isopropoxyphenyl, or 4-trifluoromethoxyphenyl.

5. A compound as recited in Claims 1 to 4, in which R³ represents phenyl, benzo[1,3]dioxol-5-yl, 2-fluorophenyl, 4-fluorophenyl, 2,4-difluorophenyl, 4-trifluoromethoxyphenyl, 2-chlorophenyl, 4-chlorophenyl, 3,4-dichlorophenyl, 4-methylphenyl, 4-isopropylphenyl, 4-t-butylphenyl, 4-cyanophenyl, 2-trifluoromethylphenyl, 4-trifluoromethylphenyl, 2-methoxyphenyl, 4-methoxyphenyl, 4-biphenyl, 4-(1-imidaznlyl)phenyl, 2-fluoro-4-methoxyphonyl, 3-fluoro-4-methoxyphenyl, 3-chloto-4-methoxyphenyl, 2-trifluoromethoxyphenyl or 4-trifluoromethoxyphenyl.

6. A compound as recited in any one of Claims 1 to 4, in which R³ represents phenyl, 2-fluorophenyl, 4-fluorophenyl, 2-chlorophenyl, 4-chlorophenyl, 4-methylphenyl, 4-isopropylphenyl, 4-cyanophenyl or 2- trifluoromethylphenyl.

7. A compound as claimed in any one of Claims 1 to 4, in which R³ represents a 4-fluorophenyl group.

8. A compound as recited in any of Claims 1 to 7, in which either m or n is 2, or both m and n are 2.

9. A compound as recited in any of Claims 1 to 8, in which Y is O or NH.

10. A compound as claimed in Claim 1, which is selected from:
1-(3,4-Dimethoxyphenethy)-4-(2-phenoxyethyl)piperazine;
1-(3-Methnxyphenethyl)-4-(2-phenoxyethyl)piperazine;
1-(4-Methoxyphenethyl)-4-)2-phenoxyethyl)piperazine;
1-(2-(Benzo[*d*][1,3]dioxol-5-yl)ethyl)-4-(2-phenoxyethyl)piperazine;
1-(3,4-Difluorophenethyl)-4-(2-phenoxyethyl)piperazine;
1-(3,4-Dimethoxyphenethyl)-4-(2-(4-chlorophenoxy)ethyl)piperazine;
4-(2-(4-(3,4-Dimethoxyphenethyl)piperazin-1-yl)ethyloxy)benzonitrile;
and pharmaceutically acceptable salts thereof.

11. A compound as claimed in Claim 1 which is selected from:
1-(4-Methoxyphenethyl)-4-(2-phenoxyethyl)piperazine dihydrochloride; and
1-(3-(Trifluoromethyl)phenethyl)-4-(2-phenoxyethyl)piperazine dihydrochloride.

12. A compound as claimed in Claim 1, which is 1-[2-(4-Fluoro-phenoxy)ethyl]-4-[3-(4-trifluoromethoxyphenyl)propyl]piperazine or a pharmaceutically acceptable salt thereof.

13. A compound as claimed in Claim 1 which is selected from:
1-[3-(3,4-Dimethoxy-phenyl)propyl]-4-(2-phenoxyethyl)piperazine
1-[3-(3,4-Dimethoxy-phenyl)propyl]-4-[2-(4-fluorophenoxy)ethyl]piperazine
1-(3-Benzo[1,3]dioxol-5-ylpropyl)-4-(2-phenoxyethyl)piperazine
1-(3-Benzo[1,3]dioxol-5-ylpropyl)-4-[2-(4-fluomphenoxy)ethyl]piperazine
1-[2-(4-Fluoro-phenoxy)ethyl]-4-[3-(4-methoxyphenyl)propyl]piperazine
1-[2-(4-Fluoro-phenoxy)ethyl]-4-[3-(4-isopropoxyphenyl)propyl]piperzine
1-[2-(4-Fluoro-phenoxy)ethyl]-4-[3-(4-trifluoromethoxyphenyl)propyl]-piperazine
1-[2-(4-Fluoro-phenoxy)ethyl]-4-[3-(3,4,5-trimethoxyphenyl)propyl]piperazine
or a pharmaceutically acceptable salt thereof.

14. A compound as claimed in Claim 1, in which m is 3.

15. A process for preparing a compound as claimed in any one of Claims 1 to 14, which comprises:
a) reducing a compound of general formula (II) 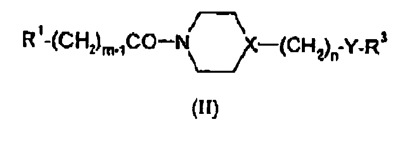 with a reducing agent;
b) for a compound of formula (I) in which X is N, reacting a compound of general formula (III) 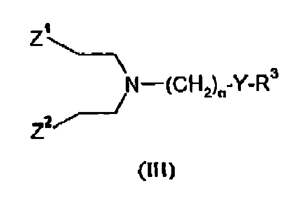 in which each of Z¹ and Z² independently represents a leaving atom or group, with a compound of general formula (IV)
R¹-(CH₂)ₘ-NH₂ (IV)
or a corresponding compounds in which one or two substituents on R¹ are protected; or
c) for a compound of formula (I) in which X is N, reacting a compound of general formula (V) 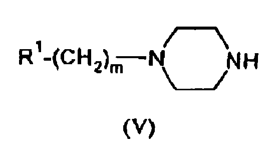 with a compound of general formula (VI)
Z³-(CH₂)ₙ-Y-R³ (VI)
in which Z³ represents a leaving atom or group;
followed by removing any protecting group and, optionally forming a pharmaceutically acceptable salt.

16. A compound of general formula (II) 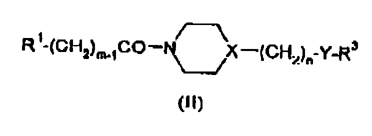 in which R¹, m, n, Y and R³ are as defined in any of Claims 1 to 14 or a salt thereof.

17. A pharmaceutical composition, which comprises a compound as claimed in any one of Claims 1-14, and a pharmaceutical acceptable diluent or carrier.

18. A compound as claimed in any one of Claims 1 to 14, for treating a condition responsive to a modulator of sigma receptor function in a patient.

19. A compound as claimed in any one of Claims 1 to 14, for use in therapy for a human or non-human subject in need thereof.

20. Use of a compound as claimed in any one of Claims 1 to 14, in the manufacture of a medicament for the treatment of a condition responsive to a modulator of sigma receptor function.

21. A compound as claimed in any one of Claims 1 to 14 for use in a method of treating a condition responsive to a modulator of sigma receptor function.

## Patentansprüche

1. Verbindung der allgemeinen Formel (I) 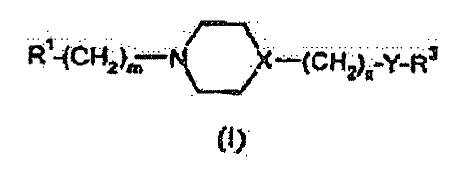 worin:
R¹ für eine Phenylgruppe, die durch einen, zwei oder drei Substituenten ausgewählt aus der Gruppe bestehend aus C₁₋₂-Alkylendioxy, einem Halogenatom,
einer Hydroxylgruppe, einer Cyanogruppe, einer C₁₋₄-Alkylgruppe, einer C₃-₆-Cycloalkylgruppe, einer Halogen-C₁₋₄-alkylgruppe, einer C₁₋₄-Alkoxygruppe und einer Halogen-C₁₋₄-alkoxygruppe substituiert ist, steht;
m für 2, 3 oder 4 steht;
X für N steht;
n für 2, 3 oder 4 steht;
Y für O oder NR² steht;
R² für Wasserstoff, C₁₋₄-Alkyl oder Phenyl-C₁₋₄-alkyl steht oder die für R³ angegebene Bedeutung besitzt und
R³ für Indan-1-yl, Indan-2-yl, 1,2,3,4-Tetrahydronaphth-1-yl oder 1,2,3,4-Tetrahydronaphth-2-yl,
wobei jede dieser Gruppen an einem nichtaromatischen Kohlenstoffatom einen Hydroxylsubstituenten tragen kann; eine C₃-₆-Cycloalkylgruppe oder eine Phenylgruppe, die unsubstituiert oder durch einen, zwei oder drei Substituenten ausgewählt aus der Gruppe bestehend aus C₁₋₂-Alkylendioxy, einem Halogenatom, einer Hydroxylgruppe, einer C₁₋₄-Alkylgruppe, einer C₃₋₆-Cycloalkylgruppe, einer Cyanogruppe, einer Phenylgruppe, einer Imidazolylgruppe, einer Halogen-C₁₋₄-alkylgruppe, einer C₁₋₉-Alkoxygruppe und einer Halogen-C₁₋₄-alkoxygruppe substituiert ist; steht;
oder ein pharmazeutisch unbedenkliches Salz davon.

2. Verbindung nach Anspruch 1, in der R¹ für Benzo-[1,3]dioxol-5-yl, 2-Fluorphenyl, 3-Fluorphenyl, 4-Fluorphenyl, 3,4-Difluorphenyl, 3-Chlorphenyl, 3-Methylphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 4-ISOpropoxyphenyl, 3,4-Dimethoxyphenyl, 2,3,4-Trimethoxyphenyl, 3,4,5-Trimethoxyphenyl, 2-Fluor-3,4-dimethoxyphenyl, 3-Chlor-4-methoxyphenyl, 4-Chlor-3-methoxyphenyl, 2-Trifluormethoxyphenyl, 4-Trifluormethoxyphenyl oder 3-Trifluormethoxyphenyl steht.

3. Verbindung nach Anspruch 1, in der R¹ für Benzo[1,3]dioxol-5-yl, 2-Fluorphenyl, 3,4-Difluorphenyl, 2-Trifluormethylphenyl, 3-Trifluormethylphenyl, 4-Trifluormethylphenyl, 2-Methoxyphenyl, 3-Methoxyphenyl, 4-Methoxyphenyl, 3,4-Dimethoxyphenyl, oder 2-Trifluormethoxyphenyl steht.

4. Verbindung nach Anspruch 1, in der R¹ für 3,4-Dimethoxyphenyl, 3,4,5-Trimethoxyphenyl, 4-Methoxyphenyl, 4-Isopropoxyphenyl oder 4-Trifluormethoxyphenyl steht.

5. Verbindung nach einem der Ansprüche 1 bis 4, in der R³ für Phenyl, Benzo[1,3]dioxol-5-yl, 2-Fluorphenyl, 4-Fluorphenyl, 2,4-Difluorphenyl, 4-Trifluormethoxyphenyl, 2-Chlorphenyl, 4-Chlorphenyl, 3,4-Dichlorphenyl, 4-Methylphenyl, 4-Isopropylphenyl, 4-t-Butylphenyl, 4-Cyanophenyl, 2-Trifluormethylphenyl, 4-Trifluormethylphenyl, 2-Methoxyphenyl, 4-Methoxyphenyl, 4-Biphenyl, 4-(1-Imidazolyl)phenyl, 2-Fluor-4-methoxyphenyl, 3-Fluor-4-methoxyphenyl, 3-Chlor-4-methoxyphenyl, 2-Trifluormethoxyphenyl oder 4-Trifluormethoxyphenyl steht.

6. Verbindung nach einem der Ansprüche 1 bis 4, in der R³ für Phenyl, 2-Fluorphenyl, 4-Fluorphenyl, 2-Chlorphenyl, 4-Chlorphenyl, 4-Methylphenyl, 4-ISOpropylphenyl, 4-Cyanophenyl oder 2-Trifluormethylphenyl steht.

7. Verbindung nach einem der Ansprüche 1 bis 4, in der R³ für eine 4-Fluorphenylgruppe steht.

8. Verbindung nach einem der Ansprüche 1 bis 7, in der entweder m oder n für 2 steht oder sowohl m als auch n für 2 stehen.

9. Verbindung nach einem der Ansprüche 1 bis 8, in der Y für O oder NH steht.

10. Verbindung nach Anspruch 1, ausgewählt unter:
1-(3,4-Dimethoxyphenethyl)-4-(2-phenoxyethyl)-piperazin;
1-(3-Methoxyphenethyl)-4-(2-phenoxyethyl)-piperazin;
1-(4-Methoxyphenethyl)-4-(2-phenoxyethyl)-piperazin;
1-(2-(Benzo[d][1,3]dioxol-5-yl)ethyl)-4-(2-phenoxyethyl)piperazin;
1-(3,4-Difluorphenethyl)-4-(2-phenoxyethyl)-piperazin;
1-(3,4-Dimethoxyphenethyl)-4-(2-(4-chlorphenoxy)-ethyl)piperazin;
4-(2-(4-(3,4-Dimethoxyphenethyl)piperazin-1-yl)-ethyloxy)benzonitril;
und pharmazeutisch unbedenklichen Salzen davon.

11. Verbindung nach Anspruch 1, ausgewählt unter:
1-(4-Methoxyphenethyl)-4-(2-phenoxyethyl)-piperazin-dihydrochlorid und
1-(3-(Trifluormethyl)phenethyl)-4-(2-phenoxyethyl)piperazin-dihydrochlorid.

12. Verbindung nach Anspruch 1, bei der es sich um 1-[2-(4-Fluorphenoxy)ethyl]-4-[3-(4-trifluormethoxyphenyl)propyl]piperazin oder ein pharmazeutisch unbedenkliches Salz davon handelt.

13. Verbindung nach Anspruch 1, ausgewählt unter:
1-[3-(3,4-Dimethoxyphenyl)propyl]-4-(2-phenoxyethyl)piperazin
1-[3-(3,4-Dimethoxyphenyl)propyl]-4-[2-(4-fluorphenoxy)ethyl]piperazin
1-(3-Benzo[1,3]dioxol-5-ylpropyl)-4-(2-phenoxyethyl)piperazin
1-(3-Benzo[1,3]dioxol-5-ylpropyl)-4-[2-(4-fluorphenoxy)ethyl]piperazin
1-[2-(4-Fluorphenoxy)ethyl]-4-[3-(4-methoxyphenyl)propyl]piperazin
1-[2-(4-Fluorphenoxy)ethyl]-4-[3-(4-isopropoxyphenyl)propyl]piperazin
1-[2-(4-Fluorphenoxy)ethyl]-4-[3-(4-trifluormethoxyphenyl)propyl]piperazin
1-[2-(4-Fluorphenoxy)ethyl]-4-[3-(3,4,5-trimethoxyphenyl)propyl]piperazin,
oder ein pharmazeutisch unbedenkliches Salz davon.

14. Verbindung nach Anspruch 1, in der m für 3 steht.

15. Verfahren zur Herstellung einer Verbindung nach einem der Ansprüche 1 bis 14, bei dem man:
a) eine Verbindung der allgemeinen Formel (II) 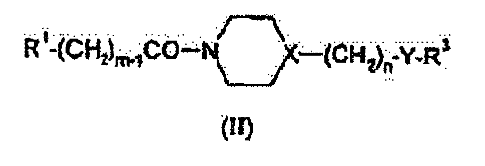 mit einem Reduktionsmittel reduziert;
b) für eine Verbindung der Formel (I), in der X für N steht, eine Verbindung der allgemeinen Formel (III) 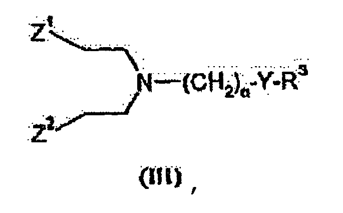 worin Z¹ und Z² jeweils unabhängig voneinander für ein Abgangsatom oder eine Abgangsgruppe stehen, mit einer Verbindung der allgemeinen Formel (IV)
**R¹⁻(CH₂)ₘ-NH₂** **(IV)**
oder einer entsprechenden Verbindung, in der ein oder beide Substituenten an R¹ geschützt sind, umsetzt; oder
c) für eine Verbindung der Formel (I), in der X für N steht, eine Verbindung der allgemeinen Formel (V) 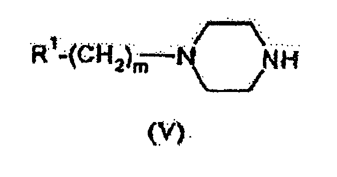 mit einer Verbindung der allgemeinen Formel (VI)
**z³-(CH₂)ₙ-Y-R³** **(VI),**
worin Z³ für ein Abgangsatom oder eine Abgangsgruppe steht, umsetzt;
danach jegliche Schutzgruppen abspaltet und gegebenenfalls ein pharmazeutisch unbedenkliches Salz bildet.

16. Verbindung der allgemeinen Formel (II) 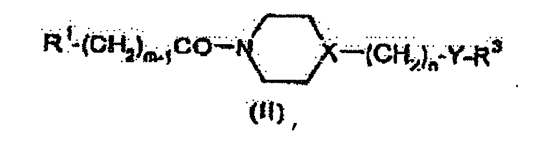 worin R¹, m, n, Y und R³ die in einem der Ansprüche 1 bis 14 angegebene Bedeutung besitzen, oder ein Salz davon.

17. Pharmazeutische Zusammensetzung, die eine Verbindung nach einem der Ansprüche 1-14 und ein pharmazeutisch unbedenkliches Verdünnungsmittel oder einen pharmazeutisch unbedenklichen Träger enthält.

18. Verbindung nach einem der Ansprüche 1 bis 14 zur Behandlung eines Leidens, das auf einen Modulator der Sigmarezeptorfunktion anspricht, bei einem Patienten.

19. Verbindung nach einem der Ansprüche 1 bis 14 zur Verwendung bei der Therapie eines menschlichen oder nichtmenschlichen Empfängers, der einer derartigen Therapie bedarf.

20. Verwendung einer Verbindung nach einem der Ansprüche 1 bis 14 bei der Herstellung eines Arzneimittels zur Behandlung eines Leidens, das auf einen Modulator der Sigmarezeptorfunktion anspricht.

21. Verbindung nach einem der Ansprüche 1 bis 14 zur Verwendung bei einem Verfahren zur Behandlung eines Leidens, das auf einen Modulator der Sigmarezeptorfunktion anspricht.

## Revendications

1. Composé de formule générale (I) 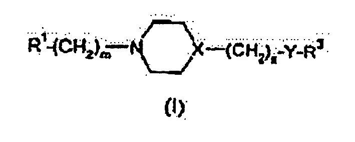 dans laquelle :
R¹ représente un groupement phényle qui est substitué par un, deux ou trois substituants choisis dans le groupe constitué de (1-2C)alkylènedioxy, d'un atome d'halogène, d'un groupement hydroxy, d'un groupement cyano, d'un groupement (1-4C)alkyle, d'un groupement (3-6C)cycloalkyle, d'un groupement halogéno(1-4C)alkyle, d'un groupement (1-4C)alcoxy, d'un groupement halogéno(1-4C)alcoxy ;
m est 2, 3 ou 4 ;
X est N ;
n est 2, 3 ou 4 ;
Y est O ou NR² _{;}
R² est hydrogène, (1-4C)alkyle ou phényl(1-4C)alkyle, ou est tel que défini pour R³ ; et
R³ représente indan-1-yle, indan-2-yle, 1,2,3,4-tétrahydronapht-1-yle, ou 1,2,3,4-tétrahydronapht-2-yle, dont chacun peut porter un substituant hydroxy sur un atome de carbone non aromatique ; un groupement (3-6C)cycloalkyle ; ou un groupement phényle qui est non substitué ou substitué par un, deux ou trois substituants choisis dans le groupe constitué de (1-2C)alkylènedioxy, d'un atome d'halogène, d'un groupement hydroxy, d'un groupement (1-4C)alkyle, d'un groupement (3-6C)cycloalkyle, d'un groupement cyano ; d'un groupement phényle, d'un groupement imidazolyle, d'un groupement halogéno(1-4C)alkyle, d'un groupement (1-4C)alcoxy et d'un groupement halogéno(1-4C)alcoxy ;
ou un sel pharmaceutiquement acceptable de celui-ci .

2. Composé selon la revendication 1, dans lequel R¹ représente benzo[1,3]dioxol-5-yle, 2-fluorophényle, 3-fluorophényle, 4-fluorophényle, 3,4-difluorophényle, 3-chlorophényle, 3-méthylphényle, 2-trifluorométhylphényle, 3-trifluorométhylphényle, 4-trifluorométhylphényle, 2-méthoxyphényle, 3-méthoxyphényle, 4-méthoxyphényle, 4-isopropoxyphényle, 3,4-diméthoxyphényle, 2,3,4-triméthoxyphényle, 3,4,5-triméthoxyphényle, 2-fluoro-3,4-diméthoxyphényle, 3-chloro-4-méthoxyphényle, 4-chloro-3-méthoxyphényle, 2-trifluorométhoxyphényle, 4-trifluométhoxyphényle, ou 3-trifluorométhoxyphényle.

3. Composé selon la revendication 1, dans lequel R¹ représente benzo[1,3]dioxol-5-yle, 2-fluorophényle, 3,4-difluorophényle, 2-trifluorométhylphényle, 3-trifluorométhylphényle, 4-trifluorométhylphényle, 2-méthoxyphényle, 3-méthoxyphényle, 4-méthoxyphényle, 3,4-diméthoxyphényle ou 2-trifluorométhoxyphényle.

4. Composé selon la revendication 1, dans lequel R¹ représente 3,4-diméthoxyphényle, 3,4,5-triméthoxyphényle, 4-méthoxyphényle, 4-isopropoxyphényle ou 4-trifluométhoxyphényle.

5. Composé selon les revendications 1 à 4, dans lequel R³ représente phényle, benzo[1,3]dioxol-5-yle, 2-fluorophényle, 4-fluorophényle, 2,4-difluorophényle, 4-trifluorométhoxyphényle, 2-chlorophényle, 4-chlorophényle, 3,4-dichlorophényle, 4-méthylphényle, 4-isopropylphényle, 4-t-butylphényle, 4-cyanophényle, 2-trifluorométhylphényle, 4-trifluorométhylphényle, 2-méthoxyphényle, 4-méthoxyphényle, 4-biphényle, 4-(1-imidazolyl)phényle, 2-fluoro-4-méthoxyphényle, 3-fluoro-4-méthoxyphényle, 3-chloro-4-méthoxyphényle, 2-trifluorométhoxyphényle ou 4-trifluorométhoxyphényle.

6. Composé selon l'une quelconque des revendications 1 à 4, dans lequel R³ représente phényle, 2-fluorophényle, 4-fluorophényle, 2-chlorophényle, 4-chlorophényle, 4-méthylphényle, 4-isopropylphényle, 4-cyanophényle ou 2-trifluorométhoxyphényle.

7. Composé selon l'une quelconque des revendications 1 à 4, dans lequel R³ représente un groupement 4-fluorophényle.

8. Composé selon l'une quelconque des revendications 1 à 7, dans lequel m ou n est 2, ou à la fois m et n sont 2.

9. Composé selon l'une quelconque des revendications 1 à 8, dans lequel Y est O ou NH.

10. Composé selon la revendication 1, qui est choisi parmi :
la 1-(3,4-diméthoxyphénéthyl)-4-(2-phénoxyéthyl)pipérazine ;
la 1-(3-méthoxyphénéthyl)-4-(2-phénoxyéthyl)pipérazine ;
la 1-(4-méthoxyphénéthyl)-4-)2-phénoxyéthyl)pipérazine ;
la 1-(2(benzo[*d*][1,3]dioxol-5-yl)éthyl)-4-(2-phénoxyéthyl)pipérazine ;
la 1-(3,4-difluorophénéthyl)-4-(2-phénoxyéthyl)pipérazine ;
la 1-(3,4-dimétoxyphénétyl)-4-(2-(4-chlorophénoxy)éthyl)pipérazine ;
le 4-(2-(4-(3,4-diméthoxyphénéthyl)pipérazin-1-yl)éthyloxy)benzonitrile ;
et les sels pharmaceutiquement acceptables de ceux-ci.

11. Composé selon la revendication 1, qui est choisi parmi :
le dichlorhydrate de 1-(4-méthoxyphénéthyl)-4-(2-phénoxyéthyl)pipérazine ;
et
le dichlorhydrate de 1-(3-(trifluorométhyl)phénéthyl)-4-(2-phénoxyéthyl)pipérazine.

12. Composé selon la revendication 1, qui est la 1-[2-(4-fluoro-phénoxy)éthyl]-4-[3-(4-trifluorométhoxyphényl)propyl]pipérazine ou un sel pharmaceutiquement acceptable de celle-ci.

13. Composé selon la revendication 1, qui est choisi parmi :
la 1-[3-(3,4-diméthoxy-phényl)propyl)-4-(2-phénoxyéthyl)pipérazine
la 1-[3-(3,4-diméthoxy-phényl)propyl)-4-[2-(4-fluorophénoxy)éthyl]pipérazine
la 1-(3-benzo[1,3]dioxol-5-ylpropyl)-4-(2-phénoxyéthyl)pipérazine
la 1-(3-benzo[1,3]dioxol-5-ylpropyl)-4-[2-(fluorophénoxy)éthyl]pipérazine
la 1-[2-(4-fluoro-phénoxy)éthyl]-4-[3-(4-méthoxyphényl)propyl]pipérazine
la 1-[2-(4-fluoro-phénoxy)éthyl]-4-[3-(4-isopropoxyphényl)propyl]pipérazine
la 1-[2-(4-fluoro-phénoxy)éthyl]-4[3-(4-trifluorométhoxyphényl)propyl]-pipérazine
la 1-[2-(4-fluoro-phénoxy)éthyl)-4-[3-(3,4,5-triméthoxyphényl)propyl]pipérazine
ou un sel pharmaceutiquement acceptable de celle-ci.

14. Composé selon la revendication 1, dans lequel m est 3.

15. Procédé de préparation d'un composé selon l'une quelconque des revendications 1 à 14, qui comprend :
a) la réduction d'un composé de formule générale (II) 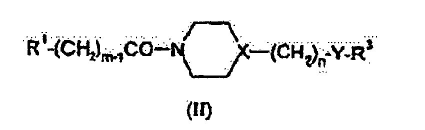 avec un agent de réduction ;
b) pour un composé de formule (I) dans laquelle X est N, la réaction d'un composé de formule générale (III) 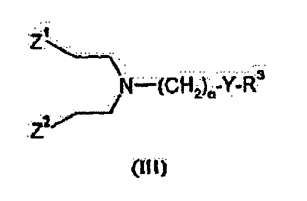 dans laquelle chacun de Z¹ et Z² représente indépendamment un atome ou un groupement partant, avec un composé de formule générale (IV)
**R¹-(CH₂)ₘ-NH₂** **(IV)**
ou un composé correspondant dans lequel un ou deux substituants sur R¹ sont protégés ; ou
c) pour un composé de formule (I) dans laquelle X est N, la réaction d'un composé de formule générale (V) 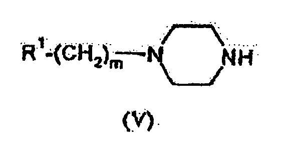 avec un composé de formule générale (VI)
**Z³-(CH₂)ₙ-Y-R³** **(VI)**
dans laquelle Z³ représente un atome ou un groupement partant ;
suivies de l'élimination de tout groupement protecteur et, éventuellement de la formation d'un sel pharmaceutiquement acceptable.

16. Composé de formule générale (II) 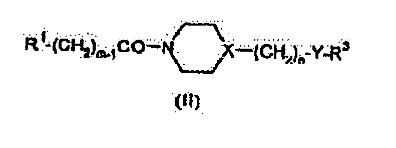 dans laquelle R¹, m, n, Y et R³ sont tels que définis dans l'une quelconque des revendications 1 à 14 ou un sel de celui-ci.

17. Composition pharmaceutique, qui comprend un composé selon l'une quelconque des revendications 1 à 14, et un diluant ou un support pharmaceutique acceptable.

18. Composé selon l'une quelconque des revendications 1 à 14, destiné au traitement d'une affection répondant à un modulateur de la fonction du récepteur sigma chez un patient.

19. Composé selon l'une quelconque des revendications 1 à 14, destiné à être utilisé en thérapie pour un sujet humain ou non humain en ayant besoin.

20. Utilisation d'un composé selon l'une quelconque des revendications 1 à 14, dans la fabrication d'un médicament destiné au traitement d'une affection répondant à un modulateur de la fonction du récepteur sigma.

21. Composé selon l'une quelconque des revendications 1 à 14, destiné à être utilisé dans une méthode de traitement d'une affection répondant à un modulateur de la fonction du récepteur sigma.
